(19) Europäisches Patentamt / European Patent Office / Office européen des brevets

(11) **EP 3 777 803 B1**

(12) **EUROPEAN PATENT SPECIFICATION**

(45) Date of publication and mention
of the grant of the patent:
**25.06.2025 Bulletin 2025/26**

(21) Application number: **19785147.0**

(22) Date of filing: **12.04.2019**

(51) International Patent Classification (IPC):
*A61F 13/53* (2006.01)    *A61F 13/15* (2006.01)
*A61F 13/534* (2006.01)    *A61L 15/24* (2006.01)
*A61L 15/60* (2006.01)

(52) Cooperative Patent Classification (CPC):
**A61F 13/15617; A61F 13/534; A61L 15/60;**
A61F 2013/530547; A61F 2013/530554;
A61F 2013/530591; A61F 2013/530788;
A61F 2013/53445; A61F 2013/5349

(86) International application number:
**PCT/JP2019/015991**

(87) International publication number:
**WO 2019/198821 (17.10.2019 Gazette 2019/42)**

(54) **WATER ABSORBENT SHEET, WATER ABSORBENT SHEET PRODUCTION METHOD, AND ABSORBENT ARTICLE**

WASSERABSORBIERENDES BLATT, HERSTELLUNGSVERFAHREN FÜR WASSERABSORBIERENDES BLATT UND ABSORBIERENDER ARTIKEL

FEUILLE ABSORBANT L'EAU, PROCÉDÉ DE PRODUCTION DE FEUILLE ABSORBANT L'EAU ET ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.04.2018  JP 2018077952**
**17.12.2018  JP 2018235117**

(43) Date of publication of application:
**17.02.2021 Bulletin 2021/07**

(73) Proprietor: **Nippon Shokubai Co., Ltd.**
**Osaka-shi, Osaka 541-0043 (JP)**

(72) Inventors:
• **TORII, Kazushi**
**Himeji-shi, Hyogo 671-1282 (JP)**
• **KITANO, Takahiro**
**Himeji-shi, Hyogo 671-1282 (JP)**
• **NODA, Yuika**
**Himeji-shi, Hyogo 671-1282 (JP)**
• **HIRAOKA, Ryuichi**
**Himeji-shi, Hyogo 671-1282 (JP)**
• **HORIMOTO, Yuichiro**
**Himeji-shi, Hyogo 671-1282 (JP)**
• **WADA, Hidenori**
**Himeji-shi, Hyogo 671-1282 (JP)**
• **HIRAUCHI, Tatsushi**
**Himeji-shi, Hyogo 671-1282 (JP)**
• **WADA, Mai**
**Himeji-shi, Hyogo 671-1282 (JP)**

(74) Representative: **Hoffmann Eitle**
**Patent- und Rechtsanwälte PartmbB**
**Arabellastraße 30**
**81925 München (DE)**

(56) References cited:
EP-A1- 3 586 957        EP-B1- 0 640 330
WO-A1-2017/170605    WO-A1-2018/155591
WO-A1-95/01146        JP-U- H0 659 039
US-A- 5 728 082        US-A1- 2017 281 422

Remarks:
The file contains technical information submitted after the application was filed and not included in this specification

EP 3 777 803 B1

## Description

Technical Field

The present invention relates to a water-absorbing sheet, a method for producing a water-absorbing sheet, and an absorbent article.

Background Art

Water-absorbing resin (super absorbent polymer; SAP) is a water-swellable, water-insoluble polymer gelling agent, whose main application is as an absorbent article for disposable diapers. An absorbent article in which water-absorbing resin and a fibrous material (e.g., pulp) are mixed is used in conventional disposable diapers. However, advanced techniques and know-how are required to appropriately mix a water-absorbing resin which is in particulate form and a bulky fibrous material, and to achieve desired performance of the absorbent body. Also, using a bulky fibrous material may be a factor that prevents a reduction in the thickness of disposable diaper products.
Therefore, recently, there are cases in which a water-absorbing sheet that has a structure in which a water-absorbing resin is sandwiched between a first sheet and a second sheet is employed as an absorbent article for disposable diapers. Since water-absorbing sheets can be produced without the use of fibrous materials that could prevent a reduction in thickness, such water-absorbing sheets have attracted attention as a member that contributes to the simplification of disposable diaper production and to a reduction in the thickness of disposable diapers.
For example, Patent Literature 1 discloses a water-absorbing sheet in which a water-absorbing layer containing water-absorbing resin is divided into a primary absorbing layer and a secondary absorbing layer. Used for the primary and secondary absorbing layers are two respective types of water-absorbing resin having differing physiological saline absorption speeds. Patent Literature 2 discloses a water-absorbing sheet using a water-absorbing resin having a fast water absorption speed in which a particle size rate index of 0.12 seconds/$\mu$m or less.

Citation List

[Patent Literature]

    [Patent Literature 1]
    Pamphlet of International Publication No. WO 2010/082373
    [Patent Literature 2]
    Pamphlet of International Publication No. WO 2011/086843

Summary of Invention

Technical Problem

For the above-described water-absorbing sheet, it is desirable to use a thin water-absorbing sheet which, with respect to urine having a wide range of salt concentrations (mass%) including 0, has both a favorable liquid absorption speed and favorable re-wet, and which has good comfortability for users, including infants, whose urine tends to change in salt concentration due to a change in the user's physical condition.

Solution to Problem

In order to solve the above problem, a water-absorbing sheet in accordance with an aspect of the present invention includes: a first sheet; a second sheet; and a particulate water-absorbing agent sandwiched between the first sheet and the second sheet, at least one sheet selected from the first sheet and the second sheet being a water-permeable sheet, the particulate water-absorbing agent including: (1) a first particulate water-absorbing agent localized in the vicinity of the first sheet; and (2) a second particulate water-absorbing agent localized in the vicinity of the second sheet, the first particulate water-absorbing agent having a gel permeation rate (GPR) which is higher than that of the second particulate water-absorbing agent, the gel permeation rate (GPR) of the first particulate water-absorbing agent differing by 10 g/min or more from that of the second particulate water-absorbing agent, the gel permeation rate (GPR) of the second particulate water-absorbing agent being 50 g/ min or less.
A water-absorbing sheet in accordance with another aspect of the present disclosure includes: two sheets; and a particulate water-absorbing agent sandwiched between the two sheets, at least one of the two sheets being a water-permeable sheet, the particulate water-absorbing agent including a particulate water-absorbing agent A and a particulate water-absorbing agent B, the particulate water-absorbing agent A adhering to a first sheet of the two sheets and the particulate water-absorbing agent B adhering to a second sheet of the two sheets when the two sheets are peeled apart, the particulate water-absorbing agent A having a gel permeation rate (GPR) which is higher than that of the particulate water-absorbing agent B, where (i) the first sheet to which the particulate water-absorbing agent A adheres is a sheet provided on a side closer to the body of a user, (ii) the second sheet to which the particulate water-absorbing agent B adheres is a sheet provided on a side farther from the body of the user, (iii) the gel permeation rate (GPR) of the particulate water-absorbing agent A differs by 10 or more from that of the particulate water-absorbing agent B, and (iv) the gel permeation rate (GPR) of the particulate water-absorbing agent B is 50 or less.

A method of producing a water-absorbing sheet in accordance with an aspect of the present invention includes at least one of the following steps: (1) dispersing a first particulate water-absorbing agent onto a first sheet; (2) dispersing a second particulate water-absorbing agent onto a second sheet; and (3) dispersing the first particulate water-absorbing agent and/or the second particulate water-absorbing agent onto an intermediate sheet, at least one sheet selected from the first sheet and the second sheet being a water-permeable sheet, the first particulate water-absorbing agent having a gel permeation rate (GPR) which is higher than that of the second particulate water-absorbing agent, the gel permeation rate (GPR) of the first particulate water-absorbing agent differing by 10 g/min or more from that of the second particulate water-absorbing agent, the gel permeation rate (GPR) of the second particulate water-absorbing agent being 50 g/min or less.

Advantageous Effects of Invention

An embodiment of the present invention provides a water-absorbing sheet which, with respect to urine having a wide range of salt concentrations (mass%) including 0, has a favorable liquid absorption speed and favorable re-wet, and which has good comfortability for users, including infants, whose urine tends to change in salt concentration due to a change in the user's physical condition.

Brief Description of Drawings

Fig. 1 is a diagram schematically illustrating a cross section of a water-absorbing sheet in accordance with an embodiment of the present invention.
Fig. 2 is a diagram schematically illustrating a cross section of a water-absorbing sheet in accordance with another embodiment of the present invention.
Fig. 3 is a two-view diagram indicating dimensions of a container used for evaluating a water-absorbing sheet in a flat state.
Fig. 4 is a diagram schematically illustrating a device 400 used for measurement of a gel permeation rate (GPR).

Description of Embodiments

[1. Definition of Terms]

[1-1. Water-absorbing sheet]

Herein, a "water-absorbing sheet" refers to a structure in which a particulate water-absorbing agent is sandwiched between two sheets. In the water-absorbing sheet, an adhesive may be used for adhesion between sheets and/or adhesion between a sheet and the particulate water-absorbing agent. A hot melt adhesive may be used for such adhesion. In addition to the particulate water-absorbing agent, the water-absorbing sheet may include other components (such as fiber components, antimicrobial agents, and/or deodorants). The water-absorbing sheet may include other sheets in addition to the two sheets sandwiching e.g. the particulate water-absorbing agent.

Ordinarily, the water-absorbing sheet is in the form of a continuous sheet or in the form of a roll in which the continuous sheet is wound. When the above water-absorbing sheet is to be used, a continuous sheet is cut into an appropriate shape (such as a rectangle) and then used as an absorbent body of e.g. a disposable diaper. In contrast, conventional disposable diapers employing high-concentration water-absorbing resin (e.g., pulp-less disposable diapers) use an individually formed absorbent body for each disposable diaper. Thus, such an absorbent body differs from the water-absorbing sheet of the present invention in the nature of the techniques involved.

[1-2. Water-absorbing resin]

Herein, the term "water-absorbing resin" refers to a polymer gelling agent having a water-swelling property (CRC), as defined in ERT 441.2-02, of 5 g/g or more, and water-insolubility (Ext), as defined in ERT 441.2-02, of 50 mass% or less.

The water-absorbing resin is preferably a hydrophilic crosslinked polymer that has been obtained by crosslinking and polymerizing unsaturated monomers each of which has a carboxyl group. Examples of forms the water-absorbing resin encompass a sheet, fibers, a film, a particulate form, and a gel. The water-absorbing sheet in accordance with an embodiment of the present invention preferably uses a particulate water-absorbing resin.

Herein, the term "water-absorbing resin" is not limited to a resin in which the total amount (100 mass%) consists of only the water-absorbing resin. The "water-absorbing resin" may be a water-absorbing resin composition containing an e.g. an additive, as long as the composition has the above-described CRC and Ext. The term "water-absorbing resin" as used herein refers to a concept also encompassing an intermediate produced in the production process of the water-absorbing resin. For example, the term "water-absorbing resin" may be used to refer to, e.g., a crosslinked hydrogel polymer produced in a polymerization step, a dried polymer produced in a drying step, and a water-absorbing resin powder which has not been subjected to surface-crosslinking.

Thus, herein, "water-absorbing resin" is a collective term used to refer not only to the water-absorbing resin itself, but also to a water-absorbing resin composition and an intermediate.

[1-3. Water-absorbing agent, particulate water-absorbing agent]

The term "water-absorbing agent" as used herein refers to an absorbent gelling agent which contains a water-absorbing resin as a main component and is for absorbing a water-based liquid. The "water-based liquid" is not particularly limited and need not consist only of water, as long as it is a liquid that contains water. The water-based liquids to be absorbed by a water-absorbing sheet according to an embodiment of the present invention are urine, menstrual blood, sweat, and other bodily fluids. In particular, the "urine" encompasses urine from e.g. infants, whose salt concentration varies widely depending on the state of health.

Herein, the term "particulate water-absorbing agent" refers to a particulate (powdered) water-absorbing agent. The concept "particulate absorbent" encompasses both a single particle of the particulate water-absorbing agent and a plurality of particles of the particulate water-absorbing agent in aggregate. Herein, "particulate" refers to a substance being in the form of one or more particles. The term "particle" refers to a relatively small piece of a material, having a size of several Å to several mm (see "particle" in "McGraw-Hill Dictionary of Scientific and Technical Terms, Third Edition", edited by editorial board of the McGraw-Hill Dictionary of Scientific and Technical Terms, Nikkan Kogyo Shimbun, 1996, p. 1929). Herein, "particulate water-absorbing agent" may be simply written as "water-absorbing agent".

The particulate water-absorbing agent contains as a main component the water-absorbing resin, which is a polymer. The particulate water-absorbing agent contains the water-absorbing resin, which is a polymer, in an amount of 60 mass% to 100 mass%, preferably 70 mass% to 100 mass%, even more preferably 80 mass% to 100 mass%, and still more preferably 90 mass% to 100 mass%. The remainder of the particulate water-absorbing agent may optionally include, for example, water and/or an additive (e.g. inorganic fine particles, polyvalent metal cations).

In other words, the upper limit of the amount of water-absorbing resin in the particulate water-absorbing agent, is, for example, 100 mass%, 99 mass%, 97 mass%, 95 mass%, or 90 mass%. The particulate water-absorbing agent preferably further includes one or more components other than the water-absorbing resin in an amount of 0 mass% to 10 mass%, in particular e.g. water and/or additives (e.g. inorganic fine particles, polyvalent metal cations).

Moisture content in the particulate water-absorbing agent is preferably 0.2 mass% to 30 mass%. As described above, the term "particulate water-absorbing agent" also encompasses a water-absorbing resin composition in which components such as water and additives are integrated with the water-absorbing resin.

Examples of the water-absorbing resin to be contained as a main component in the particulate water-absorbing agent encompass a polyacrylic acid (salt)-based resin, a polysulfonic acid (salt)-based resin, a maleic anhydride (salt)-based resin, a polyacrylamide-based resin, a polyvinyl alcohol-based resin, a polyethylene oxide-based resin, a polyaspartic acid (salt)-based resin, a polyglutamic acid (salt)-based resin, a polyalginic acid (salt)-based resin, a starch-based resin, and a cellulose-based resin. The water-absorbing resin is preferably a polyacrylic acid (salt)-based resin.

[1-4. Polyacrylic acid (salt)]

Herein, the term "polyacrylic acid (salt)" refers to polyacrylic acid and/or a salt thereof. The polyacrylic acid (salt) is a polymer which contains, as a main component, a repeating unit of acrylic acid and/or a salt thereof (hereinafter referred to as "acrylic acid (salt)") and optionally further contains a graft component. The polyacrylic acid (salt) is obtained by, for example, polymerization of acrylic acid (salt), or hydrolysis of e.g. polyacrylamide or polyacrylonitrile. Preferably, the polyacrylic acid is obtained by polymerization of acrylic acid (salt).

The wording "contains as a main component" means that the amount of the acrylic acid (salt) used when polymerizing polyacrylic acid (salt) is ordinarily 50 mol% to 100 mol%, preferably 70 mol% to 100 mol%, more preferably 90 mol% to 100 mol%, and even more preferably substantially 100 mol%, relative to a total amount of monomers for use in polymerization (excluding an internal crosslinking agent). Furthermore, the wording "includes as a main component" is also used to refer to a case where, among the repeating units contained in a polymer, the proportion of blocks derived from acrylic acid (salt) is ordinarily 50 mol% to 100 mol%, preferably 70 mol% to 100 mol%, more preferably 90 mol% to 100 mol%, and even more preferably substantially 100 mol%.

[1-5. EDANA and ERT]

The term "EDANA" is an acronym for the European Disposables and Nonwovens Associations. The term "ERT" is an acronym for EDANA Recommended Test Methods, which are European standard (substantially international standard) measuring methods for water-absorbing resin. Herein, physical properties of water-absorbing resin are measured in conformity with the 2002 version of ERT unless otherwise specified.

[1-6. Other]

Herein, a range "X to Y" means "not less than X and not more than Y".

Herein, unless otherwise specified, the unit of mass "t (ton)" means "metric ton". "Ppm" means "ppm by mass". The following term pairs are considered to be synonymous: "mass" and "weight"; "parts by mass" and "parts by weight"; "mass%" and "weight%"; and "ppm by mass" and "ppm by weight".

Herein, "... acid (salt)" means "... acid and/or a salt thereof". The term "(meth)acrylic" means "acrylic and/or methacrylic".

Herein, the unit of volume "liter" may be denoted as "l" or "L". "Mass%" may be denoted as "wt%". In measurements of trace components, values equal to or less than a detection limit are indicated as N.D. (Non Detected).

[2. Water-Absorbing Sheet]

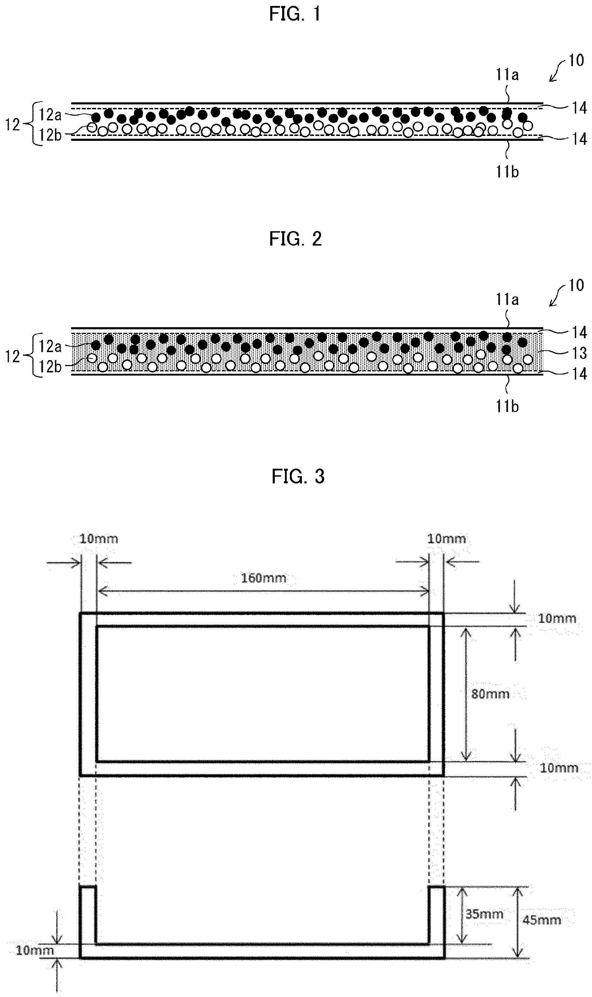

Discussed first in this section is an overview of a water-absorbing sheet in accordance with an embodiment of the present invention, with reference to Figs. 1 and 2. Thereafter is provided a discussion of e.g. each of the elements constituting the water-absorbing sheet.

Fig. 1 is a diagram schematically illustrating a cross section of a water-absorbing sheet 10 in accordance with an embodiment of the present invention. The water-absorbing sheet 10 is configured such that a particulate water-absorbing agent 12 is sandwiched between a first sheet 11a and a second sheet 11b. The particulate water-absorbing agent 12 includes a first particulate water-absorbing agent 12a localized in the vicinity of the first sheet 11a and a second particulate water-absorbing agent 12b localized in the vicinity of the second sheet 11b.

Herein, the wording "localized in the vicinity of the first sheet 11a" means that the agent is distributed in a larger amount in the vicinity of the first sheet 11a. That is, the first particulate water-absorbing agent 12a is distributed in a larger amount in the vicinity of the first sheet 11a (for example, distributed in a larger amount in a space spanning from the first sheet 11a to a thickness-wise center of the water-absorbing sheet 10). In other words, a proportion A is larger than a proportion B, where the proportion A is "a proportion of first particulate water-absorbing agent 12a among the particulate water-absorbing agent distributed in the vicinity of the first sheet 11a", and the proportion B is a "proportion of the first particulate water-absorbing agent 12a among the particulate water-absorbing agent 12 contained in the entire water-absorbing sheet 10".

As such, a configuration in which a portion of the first particulate water-absorbing agent 12a is distributed in the vicinity of the second sheet 11b does not necessarily fall outside the scope of the present invention. In other words, the first particulate water-absorbing agent 12a and the second particulate water-absorbing agent 12b need not be completely separate from each other inside the water-absorbing sheet 10. Such incomplete separation may occur, for example, when a portion of the first particulate water-absorbing agent 12a that had been dispersed, at the time of manufacturing of the water-absorbing sheet 10, so as to be distributed in the vicinity of the first sheet 11a passes through a void in an intermediate sheet 13 (such as a bulky nonwoven fabric) and moves to the vicinity of the second sheet 11b.

The above description also applies to the second sheet 11b and the second particulate water-absorbing agent 12b.

The first particulate water-absorbing agent 12a and the second particulate water-absorbing agent 12b differ from each other in terms of liquid permeability (gel permeation rate (GPR)). The gel permeation rate (GPR) of the first particulate water-absorbing agent 12a is higher than that of the second particulate water-absorbing agent. Such a configuration makes it possible for the first particulate water-absorbing agent 12a to take on the role of diffusion of liquid and for the second particulate water-absorbing agent 12b to take on the role of retention of liquid.

Note that, in the water-absorbing sheet product, the first particulate water-absorbing agent and the second particulate water-absorbing agent can be identified as follows. First, of two sheets that sandwich a particulate water-absorbing agent in a water-absorbing sheet product (for example, two sheets constituting the outermost surfaces of the water-absorbing sheet product), an arbitrarily selected sheet is considered to be a first sheet and the other a second sheet. Here, when the first sheet is peeled from the water-absorbing sheet product, the particulate water-absorbing agent adhering to the first sheet is identified as being the first particulate water-absorbing agent. Similarly, when the second sheet is peeled from the water-absorbing sheet product, the particulate water-absorbing agent adhering to the second sheet is identified as being the second particulate water-absorbing agent.

According to the identification method described above, even if a plurality of particulate water-absorbing agents are mixed at the manufacturing stage, the aggregation of particulate water-absorbing agent adhering to the first sheet and that adhering to the second sheet are identified as the first particulate water-absorbing agent and the second particulate water-absorbing agent, respectively. Furthermore, the physical properties of the first particulate water-absorbing agent and the second particulate water-absorbing agent (such as the liquid permeability) can also be measured as the respective physical properties of the aggregation of the particulate water-absorbing agent adhering to the first sheet and that adhering to the second sheet.

As described above, the determination of whether or not the configuration of the water-absorbing sheet satisfies the condition of "the first particulate water-absorbing agent is localized in the vicinity of the first sheet" can be made by determining whether or not the particulate water-absorbing agent that adheres to the first sheet when the first sheet is peeled from the water-absorbing sheet product satisfies the physical properties of the first particulate water-absorbing agent. The same applies to the determination of whether or not the condition of "the second particulate water-absorbing agent is localized in the vicinity of the second sheet" is satisfied.

[Particulate Water-Absorbing Agent A and Particulate Water-Absorbing agent B]

A water-absorbing sheet in accordance with an aspect of the present invention can also be defined as follows: a water-absorbing sheet including: two sheets; and a particulate water-absorbing agent sandwiched between the two sheets; at least one of the two sheets being a water-permeable sheet, the particulate water-absorbing agent including a particulate water-absorbing agent A and a particulate water-absorbing agent B, the particulate water-absorbing agent A adhering to a first sheet of the two sheets and the particulate water-absorbing agent B adhering to a second sheet of the two sheets when the two sheets are peeled apart, the particulate water-absorbing agent A having a gel permeation rate (GPR) which is higher than that of the particulate water-absorbing agent B, where (i) the first sheet to which the particulate water-absorbing agent A adheres is a sheet provided on a side closer to the body of a user, (ii) the second sheet to which the particulate water-absorbing agent B adheres is a sheet provided on a side farther from the body of the user, (iii) the gel permeation rate (GPR) of the particulate water-absorbing agent A differs by 10 or more from that of the particulate water-absorbing agent B, and (iv) the gel permeation rate (GPR) of the particulate water-absorbing agent B is 50 or less.

Here, "a sheet provided on a side closer to the body of the user" refers to, for example, a sheet which is provided on a side closer to a body of a wearer of a disposable diaper, the diaper employing the water-absorbing sheet as an absorbent body. This can also be expressed as "a sheet which is assumed to come into contact with a liquid earlier". The "sheet provided on the side farther from the body of the user" is a sheet that is not the above-described "sheet disposed on the side closer to the body of the user".

Note that other sheets (for example, a sheet not in contact with the particulate absorbent) may be present on either side of the "sheet disposed on the side closer to the body of the user" and the "sheet disposed on the side farther from the body of the user", or between these sheets.

In the above aspect, the particulate water-absorbing agent A corresponds to the "first particulate water-absorbing agent" described herein. In addition, the particulate water-absorbing agent B corresponds to the "second particulate water-absorbing agent" described herein. Further, among the two sheets, the sheet to which the particulate water-absorbing agent A is adhered corresponds to the "first sheet" described herein, and the sheet to which the particulate water-absorbing agent B is adhered corresponds to the "second sheet" described herein.

Thus, the following terms are interchangeable in the descriptions herein.

First particulate water-absorbing agent : particulate water-absorbing agent A
Second particulate water-absorbing agent : particulate water-absorbing agent B
First sheet : among the two sheets, the sheet to which the particulate water-absorbing agent A is adhered
Second sheet : among the two sheets, the sheet to which the particulate water-absorbing agent B is adhered.

At least one sheet selected from the first sheet 11a and the second sheet 11b is a water-permeable sheet. It is preferable that the water-permeable sheet is the sheet on a side that comes into contact with the liquid when the water-absorbing sheet 10 is used (for example, a side closer to the body of the user when the water-absorbing sheet 10 is used as an absorbent body of a disposable diaper). Further, considering the difference in gel permeation rate (GPR) between the first particulate water-absorbing agent 12a and the second particulate water-absorbing agent 12b, it is preferable that the first sheet be a water-permeable sheet, and more preferable that only the first sheet be a water-permeable sheet.

Such a configuration enables the water-absorbing sheet 10 to exhibit sufficient performance (liquid absorption speed, re-wet, and the like). This is because of the following mechanism. Configuring the first sheet 11a to be a water-permeable sheet causes liquid that has come in contact with the water-absorbing sheet 10 to quickly move into the layer of the particulate water-absorbing agent 12. The liquid that has moved to the layer of the particulate water-absorbing agent 12 is first quickly diffused in the layer of the first particulate water-absorbing agent 12a. This is because the first particulate water-absorbing agent 12a has a high gel permeation rate (GPR). Next, the diffused liquid is retained by the second particulate water-absorbing agent 12b and is kept inside the water-absorbing sheet 10. This is because the second particulate water-absorbing agent 12b has a low gel permeation rate (GPR).

In order for the water-absorbing sheet 10 to exhibit the above-described function with respect to urine having a wide range of salt concentrations including 0, a configuration can be employed in which (1) the difference between the gel permeation rates (GPRs) of the first particulate water-absorbing agent 12a and the second particulate water-absorbing agent 12b is set to 10 g /min or more, and (2) the gel permeation rate (GPR) of the second particulate water-absorbing agent is set to 50 g/min or less.

The water-absorbing sheet 10 may include a sheet in addition to the first sheet 11a and the second sheet 11b. For example, an intermediate sheet 13 may be provided between the first sheet 11a and the second sheet 11b, as illustrated in Fig. 2. The intermediate sheet 13 makes it easier to localize the first particulate water-absorbing agent 12a and the second particulate water-absorbing agent 12b in the vicinity of the first sheet 11a and the second sheet 11b, respectively, as illustrated in Fig. 2. Further, if a bulky material (preferably, for example, a polypropylene nonwoven fabric having a thickness of 3 mm to 7 mm without load, and a mass per unit area of 40 g/m$^2$ to 60 g/m$^2$) is used as the intermediate sheet 13, the particulate water-absorbing agent 12 can be fixed by being entangled in the intermediate sheet 13. This makes it possible to localize the first particulate water-absorbing agent 12a and the second particulate water-absorbing agent 12b to the vicinity of the first sheet 11a and the second sheet 11b, respectively, in a preferable manner.

In order to localize the first particulate water-absorbing agent 12a and the second particulate water-absorbing agent 12b to the vicinity of the first sheet 11a and the second sheet 11b, respectively, it is preferable to use an adhesive 14. In other words, during the process of manufacturing the water-absorbing sheet 10, providing a layer of the adhesive 14 when dispersing the particulate water-absorbing agent onto the sheets provided in the water-absorbing sheet 10 (onto the first sheet 11a, the second sheet 11b, and/or the intermediate sheet 13), makes it possible to fix the particulate water-absorbing to those sheets (see Figs. 1 and 2). This makes it possible to localize the first particulate water-absorbing agent 12a to the vicinity of the first sheet 11a and the second particulate water-absorbing agent 12b to the vicinity of the second sheet 11b, in a more preferable manner. A method of producing a water-absorbing sheet using an adhesive is described in detail in section [2-5].

In addition, the water-absorbing sheet 10 may have a region in which at least one selected from the first particulate water-absorbing agent 12a and the second particulate water-absorbing agent 12b is absent, the region extending in a lengthwise direction (for details, see section [2-4]).

[2-1. Sheets]

The first sheet and the second sheet are not particularly limited in terms of the form and materials thereof, as long as the first sheet and second sheet make it possible to sandwich and fix the particulate water-absorbing agent. Similarly, the form and materials of the intermediate sheet are not particularly limited.

The above sheets have a tensile strength of ordinarily 20 kgf/5cm (200 N/5cm) or more, preferably 30 kgf/5cm (300 N/5cm) or more, and more preferably 50 kgf/5cm (500 N/5cm) or more. At least one sheet has such tensile strength. Preferably at least one sheet selected from the first sheet and the second sheet has such tensile strength. More preferably, both the first sheet and the second sheet have such tensile strength.

Regarding the thickness of the sheets, a thinner sheet is more preferable, provided that the sheet has the above tensile strength. Each of the sheets has a thickness of ordinarily 0.01 mm to 2 mm, preferably 0.02 mm to 1 mm, more preferably 0.03 mm to 0.6 mm, and even more preferably from 0.05 mm to 0.5 mm.

Each of the sheets has a mass per unit area of ordinarily 5 g/m$^2$ to 300 g/m$^2$, preferably 8 g/m$^2$ to 200 g/m$^2$, more preferably 10 g/m$^2$ to 100 g/m$^2$, and even more preferably 11 g/m$^2$ to 50 g/m$^2$.

(Water-Permeable Sheet)

The first sheet, the second sheet, and the intermediate sheet used in the water-absorbing sheet in accordance with an embodiment of the present invention may each be a water-permeable sheet having water permeability, and may each be of the same kind or of different kinds. The permeability coefficient (JIS A 1218:2009) of each water-permeable sheet is ordinarily $1 \times 10^{-5}$ cm/sec or more, preferably $1 \times 10^{-4}$ cm/sec or more, more preferably $1 \times 10^{-3}$ cm/sec or more, even more preferably $1 \times 10^{-2}$ cm/sec or more, and most preferably $1 \times 10^{-1}$ cm/sec or more.

(Material of Sheets)

For the material of the first sheet, the second sheet, and the intermediate sheet used in the water-absorbing sheet in accordance with an embodiment of the present invention, a nonwoven fabric is preferable. The material of the nonwoven fabric is not particularly limited, but from the viewpoint of liquid permeability, flexibility, and strength of the water-absorbing sheet, preferable examples include the following: polyolefin fibers (such as polyethylene (PE) and polypropylene (PP)), polyester fibers (such as polyethylene terephthalate (PET), polytrimethylene terephthalate (PTT), and polyethylene naphthalate (PEN)), polyamide fibers (such as nylon), rayon fibers, and pulp (such as cellulose) fibers. Also preferable are nonwoven fabrics of other synthetic fibers, and nonwoven fabrics produced by mixing synthetic fibers with e.g. cotton, silk, hemp, and/or pulp (cellulose) fibers.

The nonwoven fabric described above may contain only one kind of the above described fibers or may contain a combination of two or more kinds of fibers.

The nonwoven fabric used for the intermediate sheet is preferably an air-through nonwoven fabric. The nonwoven fabric used for the intermediate sheets is preferably a bulky nonwoven fabric, specifically, preferably a nonwoven fabric having both a mass per unit area of 30 g/m$^2$ or more and a thickness of 1 mm or more.

The nonwoven fabric may include a small amount of pulp fibers to an extent that the thickness of the water-absorbing sheet is not increased.

(Hydrophilicity of Nonwoven Fabric)

Herein, the "hydrophilicity of nonwoven fabric" is measured by the method described in the JAPAN TAPPI paper and pulp test method no.68 "Paper and paper board - water repellency test method" (for details, see "JAPAN TAPPI Paper and Pulp Test Methods 2000 Edition," edited by Japan Technical Association of the Pulp and Paper Industry, published 2001 by the Japan Technical Association of the Paper and Pulp Technical Association).

The hydrophilicity of the nonwoven fabric used in the water-absorbing sheet in accordance with an embodiment of the present invention is ordinarily 5 to 200, preferably 8 to 150, more preferably 10 to 100, and even more preferably 12 to 80.

If the hydrophilicity of the nonwoven fabric is too low, the liquid absorption performance of the water-absorbing sheet will deteriorate. Conversely, if the hydrophilicity of the nonwoven fabric is too high, the liquid absorption performance will not improve accordingly. As such, it is preferable that the hydrophilicity of the nonwoven fabric be within the range described above.

Among the above-described fibers, examples of the material of the nonwoven fabric having such hydrophilicity encompass rayon fibers. Further, the material of the nonwoven fabric may be a material obtained by subjecting a hydrophobic synthetic fiber such as a polyolefin fiber or a polyester fiber to a hydrophilization treatment by a known method, to impart an appropriate hydrophilicity.

(Hydrophilization Treatment)

As described above, the nonwoven fabric used in the water-absorbing sheet in accordance with an embodiment of the present invention is preferably a hydrophilic nonwoven fabric in order to enhance water permeability, but a hydrophilization agent (such as a surfactant) may be used to hydrophilize a nonwoven fabric or fibers which are a material of a nonwoven fabric.

Examples of hydrophilization agents encompass anionic surfactants (such as aliphatic sulfonate and sulfate ester salt of higher alcohol), cationic surfactants (such as quaternary ammonium salt), nonionic surfactants (such as polyethylene glycol fatty acid ester, polyglyceryl fatty acid ester, and sorbitan fatty acid ester), silicone-based surfactants (such as polyoxyalkylene-modified silicon), and stain-release agents such as those containing polyester-based resin, polyamide-based resin, acrylic resin, and urethane-based resin.

[2-2. Particulate Water-Absorbing Agent]

(Gel Permeation Rate (GPR); Liquid Permeability)

Herein, the "liquid permeability" of a particulate water-absorbing agent or a water-absorbing resin refers to the flowability of a liquid passing between swollen gel particles under load. Gel permeation rate (GPR) is used as an index of this.

A water-absorbing sheet in accordance with an embodiment of the present invention includes the first particulate water-absorbing agent localized in the vicinity of the first sheet, and the second particulate water-absorbing agent localized in the vicinity of the second sheet. The first particulate water-absorbing agent and the second particulate water-absorbing agent differ from each other in terms of liquid permeability (gel permeation rate (GPR)). The first particulate water-absorbing agent has a high gel permeation rate (GPR) and the second particulate water-absorbing agent has a low gel permeation rate (GPR).

In order to bring about an effect of the present invention, (i.e., to provide a water-absorbing sheet which, with respect to urine having a wide range of salt concentrations (mass%) including 0, has a favorable liquid absorption speed and favorable re-wet, and which has good comfortability for users, including infants, whose urine tends to change in salt concentration due to a change in the user's physical condition), the gel permeation rates (GPRs) of the first particulate water-absorbing agent and the second particulate water-absorbing agent are either necessarily or preferably as follows.

The difference between the gel permeation rates (GPRs) of the first particulate water-absorbing agent and the second particulate water-absorbing agent is 10 g/min or more, preferably 15 or more, more preferably 20 or more, even more preferably 30 or more, even more preferably 40 or more, and particularly preferably 50 or more. The upper limit value of the difference between the gel permeation rates (GPRs) of the first particulate water-absorbing agent and the second particulate water-absorbing agent may be set to about 1000, preferably about 500, and more preferably about 300, in order to prevent the occurrence of leakage due to the excessively high diffusivity.

The gel permeation rate (GPR) in g/min of the second particulate water-absorbing agent is 50 or less, preferably 40 or less, more preferably 30 or less, even more preferably 20 or less, and even more preferably 10 or less. The lower limit value of the gel permeation rate (GPR) of the second particulate water-absorbing agent is 0.

The gel permeation rate (GPR) of the first particulate water-absorbing agent is not particularly limited as long as the conditions described above are satisfied (the difference between the gel permeation rates (GPRs) of the first particulate water-absorbing agent and second particulate water-absorbing agent, and conditions described for the gel permeation rate (GPR) of the second particulate water-absorbing agent). As such, the lower limit value of the gel permeation rate (GPR) in g/min of the first particulate water-absorbing agent is 10 or more, preferably 20 or more, and more preferably 30 or more. The upper limit value of the gel permeation rate (GPR) of the first particulate water-absorbing agent is ordinarily 1000 or less, preferably 800 or less, more preferably 600 or less, even more preferably 400 or less, and particularly preferably 300 or less, because there is a limit to measurements of gel permeation rate (GPR).

The above-described liquid permeability may be achieved by one type of particulate water-absorbing agent or may be achieved by a mixture of two or more types of the particulate water-absorbing agent. In other words, the first particulate water-absorbing agent and/or the second particulate water-absorbing agent are not limited to being a single type of particulate water-absorbing agent, and may each be a mixture of a plurality of types of particulate water-absorbing agent.

As described above, the particulate water-absorbing agent (particularly, the first particulate water-absorbing agent) contained in the water-absorbing sheet in accordance with an embodiment of the present invention has a relatively high gel permeation rate (GPR). A particulate water-absorbing agent having such a high gel permeation rate (GPR) can be easily obtained by aqueous solution polymerization and ordinarily cannot be obtained by reversed phase suspension polymerization. As such, it may be impossible to obtain a desired gel permeation rate (GPR) if the particulate water-absorbing agent (particularly, the first particulate water-absorbing agent) included in the water-absorbing sheet in accordance with an embodiment of the present invention contains an excessive amount of a particulate water-absorbing agent obtained by reversed phase suspension polymerization. For this reason, when a particulate water-absorbing agent obtained by reversed phase suspension polymerization is used, it is preferable to use such a water-absorbing agent for the purpose of improving a physical property other than the gel permeation rate (GPR), and in an amount at which a desired gel permeation rate (GPR) can be obtained.

(Used Amount of Particulate Water-Absorbing Agent)

The amount of the particulate water-absorbing agent contained per unit area in the water-absorbing sheet according to an embodiment of the present invention is ordinarily 80 g/m$^2$ to 1200 g/m$^2$, preferably 100 g/m$^2$ to 1000 g/m$^2$, more preferably 150 g/m$^2$ to 900 g/m$^2$, even more preferably 200 g/m$^2$ to 800 g/m$^2$, and even more preferably 220 g/m$^2$ to 700 g/m$^2$.

Setting the amount of the particulate water-absorbing agent to fall within the above range enables the water-absorbing sheet to bring about an effect of the present invention. If the amount used of the particulate water-absorbing agent is too small, the water absorbing ability of the water-absorbing sheet may be insufficient. Conversely, if the amount of the particulate water-absorbing agent is too large, a problem may arise in terms of hygiene and air permeability of the absorbent article using the water-absorbing sheet.

(Arrangement of Particulate Water-Absorbing Agent)

The water-absorbing sheet in accordance with an embodiment of the present invention has at least two or more layers of the particulate water-absorbing agent.

Layers of the particulate water-absorbing agent may be divided from each other by further including one or more intermediate sheets between the first sheet and the second sheet. In such a configuration, the first particulate water-absorbing agent and the second particulate water-absorbing agent are provided so as to be on opposite sides of the intermediate sheet.

In addition to the first particulate water-absorbing agent and the second particulate water-absorbing agent, one or more further layers of the particulate water-absorbing agent may be provided. Further, in addition to the particulate water-absorbing agent, some other third component (such as an antimicrobial agent and/or a deodorant) may be provided.

In a configuration in which the water-absorbing sheet in accordance with an embodiment of the present invention has a bilayer structure (that is, a layer of a first particulate water-absorbing agent and a layer of a second particulate water-absorbing agent), the mass ratio of the first particulate water-absorbing agent to the second particulate water-absorbing agent is preferably 5/95 to 50/50, more preferably 5/95 to 40/60, even more preferably 5/95 to 30/70, and even more preferably 5/95 to 20/80. Setting the mass ratio of the first particulate water-absorbing agent to the second particulate water-absorbing agent to fall in the range of 5/95 to 50/50 makes it possible to bring about an effect of the present invention, i.e., to provide a water-absorbing sheet which, with respect to urine having a wide range of salt concentrations (mass%) including 0, has a favorable liquid absorption speed and favorable re-wet, and which has good comfortability for users, including infants, whose urine tends to change in salt concentration due to a change in the user's physical condition.

[2-3. Adhesive]

In the water-absorbing sheet in accordance with an embodiment of the present invention, the method of fixing the sheets to each other and the method of fixing the sheets and the particulate water-absorbing agent to each other are not particularly limited. Possible examples encompass a method of fixing by pressure bonding, methods using various binders, a method of melting and heat sealing the material of a sheet itself. However, a method of fixing by using an adhesive is preferable.

The adhesive used for fixing is not particularly limited. However, hot melt adhesives are preferable over solution-type adhesives. This is because a hot melt adhesive does not use a solvent and therefore improves productivity by avoiding the need to remove the solvent, while also avoiding the risk of the solvent remaining.

When a hot melt adhesive is used for fixing, a sheet or a particulate water-absorbing agent may be prepared such that the surface of the sheet or particulate water-absorbing agent contains a hot melt adhesive in advance. Alternatively, the hot melt adhesive may be used in the production process of the water-absorbing sheet.

The form and melting point of the hot melt adhesive can be selected as appropriate. The hot melt adhesive may have the form of particles, fibers, a net, or a film, or may have the form of a heat-melted liquid. When a hot melt adhesive is used in the form of particles, the particle diameter thereof is approximately 0.01 times to 2 times, preferably approximately 0.02 times to 1 time, and more preferably approximately 0.05 times to 0.5 times the average particle diameter of the particulate water-absorbing agent. The melting point (or softening point) of the hot melt adhesive is ordinarily 50°C to 200°C, and preferably 60°C to 180°C.

The type of the hot melt adhesive used in the water-absorbing sheet in accordance with an embodiment of the present invention is not particularly limited. Preferable examples that can be used encompass an ethylene-vinyl acetate copolymer adhesive, a styrene-based elastomer adhesive, a polyolefin-based adhesive, and a polyester-based adhesive. One kind of hot melt adhesive may be used alone, or two or more kinds may be used in combination.

Examples of the ethylene-vinyl acetate copolymer adhesive encompass an ethylene-vinyl acetate copolymer (EVA) adhesive, an ethylene-ethyl acrylate copolymer (EEA), and an ethylene-butyl acrylate copolymer (EBA). Examples of the styrene-based elastomer adhesive encompass a styrene-isoprene block copolymer (SIS) adhesive, a styrene-butadiene block copolymer (SBS) adhesive, a styrene-isobutylene block copolymer (SIBS) adhesive, and a styrene-ethylene-butylene-styrene block copolymer (SEBS) adhesive. Examples of the polyolefin-based adhesive encompass a polyethylene adhesive, a polypropylene adhesive, and an atactic polypropylene adhesive. Examples of the polyester-based adhesive (such as a copolymerized polyolefin adhesive) encompass a polyethylene terephthalate (PET) adhesive, a polybutylene terephthalate (PBT) adhesive, and a copolymerized polyester adhesive.

In the water-absorbing sheet in accordance with an embodiment of the present invention, the contained amount (mass) of the adhesive (hot melt adhesive) is more than 0 times to 1.0 time, preferably 0.1 times to 1.0 time, more preferably 0.11 times to 0.50 times, even more preferably 0.12 times to 0.30 times, and even more preferably 0.14 times to 0.25 times the contained amount (mass) of the water-absorbing resin. An excessive contained amount of the adhesive is disadvantageous as it leads to an increase in manufacturing costs and an increase in mass of the water-absorbing sheet. Further, an excessive contained amount of the adhesive limits the swelling of the particulate water-absorbing agent and therefore may cause a decrease in the water absorbing ability of the water-absorbing sheet.

[2-4. Form and Performance of Water-Absorbing Sheet]

The water-absorbing sheet in accordance with an embodiment of the present invention can be made thinner than an absorbent body used in a conventional absorbent article. When the above water-absorbing sheet is used for a disposable diaper, its thickness in a dry state is 5 mm or less, preferably 4 mm or less, more preferably 3 mm or less, and even more preferably 2 mm or less. The lower limit of this thickness is 0.2 mm or more, preferably 0.3 mm or more, and more preferably 0.5 mm or more, in view of the strength of the water-absorbing sheet and the diameter of the particulate water-absorbing agent.

In order to further impart e.g. liquid permeability, diffusivity, and flexibility to the water-absorbing sheet, the surface of the water-absorbing sheet (that is, the outer surface side of the first sheet and/or the second sheet) may be subjected to embossing as appropriate. The region to be subjected to embossing may be the entire surface of the water-absorbing sheet, or a part of the surface of the water-absorbing sheet. Providing a continuous embossed region in the lengthwise direction of the water-absorbing sheet allows liquid to be easily diffused in the lengthwise direction.

In addition, the particulate water-absorbing agent may be dispersed over the entire surface of the water-absorbing sheet (that is, the entire surface on the inner surface side of the first sheet and/or the second sheet), or a region in which the particulate water-absorbing agent absent may be provided to part of the water-absorbing sheet. When a region in which the particulate water-absorbing agent is absent is provided, it is preferable that the region is provided in a channel-like shape (stripe) in the lengthwise direction of the water-absorbing sheet.

Thusly providing the embossed region and/or the region in which the particulate water-absorbing agent is absent, in a continuous manner in the lengthwise direction, allows such regions to serve as a passage (liquid transport passage) for flow of a large amount of liquid. The embossed region and/or the region in which the particulate water-absorbing agent is absent may be straight, curved, or corrugated.

The peel strength of the water-absorbing sheet in accordance with an embodiment of the present invention is ordinarily 0.05 N/7cm to 3.0 N/7cm, preferably 0.1 N/7cm to 2.5 N/7cm, more preferably 0.15 N/7cm to 2.0 N/7cm, and even more preferably 0.2 N/7cm to 1.5 N/7cm. If the peel strength of the water-absorbing sheet exceeds 3.0 N/7cm, the adhesive strength becomes too strong and the particulate water-absorbing agent used may not exhibit sufficient water absorption performance.

[2-5. Method of Producing Water-Absorbing Sheet]

A method of producing a water-absorbing sheet in accordance with an embodiment of the present invention includes at least one of the following steps: (1) a step of dispersing the first particulate water-absorbing agent onto the first sheet, (2) a step of dispersing the second particulate water-absorbing agent onto the second sheet, and (3) a step of dispersing the first particulate water-absorbing agent and/or the second particulate water-absorbing agent onto the intermediate sheet. The conditions which the first particulate water-absorbing agent and the second particulate water-absorbing agent satisfy are as described above. More specific examples of this production method encompass the following methods (a) to (c).

(a) The first particulate water-absorbing agent (and, preferably, the adhesive) is uniformly dispersed onto the first sheet. A similar operation is performed for the second sheet and the second particulate water-absorbing agent. The first sheet and the second sheet are superimposed so that the respective faces onto which the particulate water-absorbing agent was dispersed are facing each other, and then the first sheet and second sheet are pressure bonded. The pressure bonding is preferably thermocompression bonding carried out at around the melting temperature of the adhesive.

(b) The first particulate water-absorbing agent (and, preferably, the adhesive) is dispersed onto the first sheet and then passed through a furnace so as to be fixed to such an extent that the first particulate water-absorbing agent is not scattered. A similar operation is performed for the second sheet and the second particulate water-absorbing agent. The first sheet and the second sheet are superimposed so that the respective faces onto which the particulate water-absorbing agent was dispersed are facing each other, and then the first sheet and second sheet are subjected to thermocompression bonding.

(c) The adhesive is melted and applied to the first sheet, and thereafter the particulate water-absorbing agent is uniformly dispersed onto the first sheet to form a layer. A similar operation is performed for the second sheet and the second particulate water-absorbing agent. The first sheet and the second sheet are superimposed so that the respective faces onto which the particulate water-absorbing agent was dispersed are facing each other, and then the first sheet and second sheet are pressure bonded with use of e.g. a roll press machine.

Among these methods, the methods (a) and (c) are preferable from the viewpoint of their simplicity and high production efficiency. Note that it is also possible to produce a water-absorbing sheet by any combination of the methods (a) through (c).

Examples of a method of producing a water-absorbing sheet including an intermediate sheet encompass the following methods (d) to (g).

(d) The first particulate water-absorbing agent (and, preferably, the adhesive) is uniformly dispersed onto the first sheet. An intermediate sheet is superimposed thereon and pressure bonded. Furthermore, the second particulate water-absorbing agent (and, preferably, the adhesive) is uniformly dispersed onto the surface of the intermediate sheet on the side facing away from the first particulate water-absorbing agent. A second sheet is superimposed thereon and pressure bonded (preferably under heated conditions in which the hot melt adhesive melts, or in a state in which the hot melt adhesive is already melted).

(e) The second particulate water-absorbing agent is uniformly dispersed onto the intermediate sheet. The adhesive is dispersed onto the second sheet. Then, the surface of the intermediate sheet on which the second particulate water-absorbing agent has been dispersed and the surface of the second sheet on which the adhesive has been dispersed are pressure bonded. Next, after this pressure bonding, the first particulate water-absorbing agent is uniformly dispersed onto the surface of the intermediate sheet which faces away from the surface on which the second particulate water-absorbing agent has been dispersed. The adhesive is dispersed onto the first sheet. Then, the surface of the intermediate sheet onto which the first particulate water-absorbing agent has been dispersed and the surface of the first sheet onto which the adhesive has been dispersed are pressure bonded. The pressure bonding is preferably carried out under heated conditions in which the hot melt adhesive melts, or in a state in which the hot melt adhesive is already melted.

(f) The adhesive is dispersed onto the second sheet. Next, the second particulate water-absorbing agent is uniformly dispersed thereon. Next, the intermediate sheet is placed thereon and pressure bonded. Next, the adhesive is dispersed onto a surface of the intermediate sheet which faces away from the second particulate water-absorbing agent. Next, the first particulate water-absorbing agent is uniformly dispersed thereon. Next, the first sheet is placed thereon and pressure bonded. The pressure bonding is preferably carried out under heated conditions in which the hot melt adhesive melts, or in a state in which the hot melt adhesive is already melted.

(g) The adhesive is dispersed onto the second sheet. Next, the second particulate water-absorbing agent is uniformly dispersed thereon. Next, the intermediate sheet is placed thereon and pressure bonded. Next, the first particulate water-absorbing agent is dispersed uniformly onto a surface of the intermediate sheet which faces away from the second particulate water-absorbing agent. The adhesive is dispersed onto the first sheet. Then, the surface of the intermediate sheet onto which the first particulate water-absorbing agent has been dispersed and the surface of the first sheet onto which the adhesive has been dispersed are pressure bonded. The pressure bonding is preferably carried out under heated conditions in which the hot melt adhesive melts, or in a state in which the hot melt adhesive is already melted.

As a step other than those described above, a step of subjecting the water-absorbing sheet to embossing may be carried out for the purpose of improving the texture of the water-absorbing sheet and improving liquid absorption performance. Embossing may be carried out simultaneously with the pressure bonding of the first sheet and the second sheet, or may be carried out after the water-absorbing sheet has been produced.

In the method of producing the water-absorbing sheet in accordance with an embodiment of the present invention, an additive (such as a deodorant, fibers, an antimicrobial agent, and/or a gel stabilizer) may be added as appropriate. The amount of the additive to be added is 0 mass% to 100 mass%, preferably 0 mass% to 50 mass%, and more preferably 0 mass% to 10 mass%, relative to the mass of the particulate water-absorbing agent. The above production method may use a particulate water-absorbing agent which has been mixed in advance with the additive, or the additive may be added during the production process.

The dimensions of the water-absorbing sheet to be produced may be set as appropriate. Ordinarily, the width is 3 m to 10 m, and the length is several tens of meters to several thousands of meters (in the form of a continuous sheet or roll). The water-absorbing sheet produced is used after being cut according to its purpose (according to the size of the absorbent body to be used).

In addition to the above examples, examples of methods of producing the water-absorbing sheet encompass the methods disclosed in the following Patent Literature: International Publication No. WO 2012/174026, International Publication No. WO 2013/078109, International Publication No. WO 2015/041784, International Publication No. WO 2011/117187, International Publication No. WO 2012/001117, International Publication No. WO 2012/024445, International Publication No. WO 2010/004894, International Publication No. WO 2010/004895, International Publication No. WO 2010/076857, International Publication No. WO 2010/082373, International Publication No. WO 2010/113754, International Publication No. WO 2010/143635, International Publication No. WO 2011/043256, International Publication No. WO 2011/086841, International Publication No. WO 2011/086842, International Publication No. WO 2011/086843, International Publication No. WO 2011/086844, International Publication No. WO 2011/117997, International Publication No. WO 2011/118409, International Publication No. WO 2011/136087, International Publication No. WO 2012/043546, International Publication No. WO 2013/099634, International Publication No. WO 2013/099635, Japanese Patent Application Publication, Tokukai, No. 2010-115406, Japanese Patent Application Publication, Tokukai, No. 2002-345883, Japanese Patent Application Publication, Tokukaihei, No. 6-315501, Japanese Patent Application Publication, Tokukaihei, No. 6-190003, Japanese Patent Application Publication, Tokukaihei, No. 6-190002, Japanese Patent Application Publication, Tokukaihei, No. 6-190001, Japanese Patent Application Publication, Tokukaihei, No. 2-252558, Japanese Patent Application Publication, Tokukaihei, No. 2-252560, and Japanese Patent Application Publication, Tokukaihei, No. 2-252561. The methods of producing a water-absorbing sheet disclosed in the above literature can also be referred to as appropriate.

[3. Absorbent Article]

An absorbent article in accordance with an embodiment of the present invention has a structure in which the water-absorbing sheet described in section [2] above is sandwiched by a liquid-permeable sheet and a liquid-impermeable sheet. Specific examples of the absorbent article encompass a disposable diaper, an incontinence pad, a sanitary napkin, a pet sheet, a drip sheet for food, and a waterproofing agent for power cables.

As the liquid-permeable sheet and the liquid-impermeable sheet, sheets that are publicly known in the technical field of absorbent articles can be used without any particular limitation. The absorbent article can be produced by a publicly known method.

The absorbent article in accordance with an embodiment of the present invention is preferably configured such that (1) the first sheet is a water-permeable sheet and (2) the first sheet is provided on a side toward the liquid-permeable sheet.

With such a configuration, the liquid comes into contact with the first particulate water-absorbing resin (high gel permeation rate (GPR)) before coming into contact with the second particulate the water-absorbing resin (low gel permeation rate (GPR)). Therefore, the mechanism described in section [2] makes it possible to sufficiently achieve the performance (such as liquid absorption speed and re-wet) of the water-absorbing sheet, which is an effect of the present invention.

[4. Physical Properties of Particulate Water-Absorbing Agent]

The water-absorbing sheet in accordance with an embodiment of the present invention includes the first particulate water-absorbing agent and the second particulate water-absorbing agent. These particulate water-absorbing agents preferably have the physical properties described below. Note that, unless otherwise stated, when the first particulate water-absorbing agent and/or the second particulate water-absorbing agent is a mixture of a plurality of types of particulate water-absorbing agent, the following discussion is describes the physical properties of the mixture.

[4-1. Gel Permeation Rate (GPR); Liquid Permeability]

(Method of Measuring Gel Permeation Rate (GPR))

The gel permeation rate (GPR) of the particulate water-absorbing agent (the first particulate water-absorbing agent and the second particulate water-absorbing agent) contained in the water-absorbing sheet in accordance with an embodiment of the present invention can be measured by the following procedure, with reference to the saline flow conductivity (SFC) test described in the specification of US Patent No. 5849405, and with changes made to the measurement conditions.

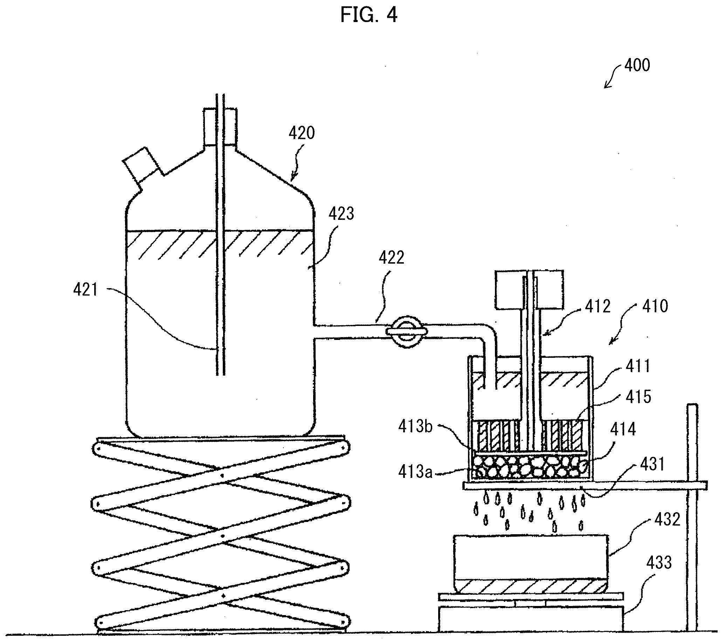

A device 400 as illustrated in Fig. 4 is used as a device for measurement. The configuration of the device 400 can be divided generally into a container 410 and a tank 420.

The container 410 includes a cell 411 (inner diameter: 6 cm). The cell 411 can house a swollen gel 414 (obtained by causing the particulate water-absorbing agent to absorb water). A liquid 423 can be introduced into the cell 411. Further, fitting a piston 412 to the cell 411 makes it possible to apply pressure to the swollen gel 414. Metal gauzes 413a and 413b (no. 400 stainless-steel gauze, mesh size: 38 $\mu$m) are provided at the bottom surface of the cell 411 and the bottom surface of the piston 412, and the swollen gel 414 (and the particulate water-absorbing agent) cannot pass therethrough. Used as the liquid 423 is a 0.90 mass% aqueous sodium chloride solution.

The "deionized water" is a concept including ultrapure water. Herein, used as the deionized water is water having a conductivity (25°C) of 1.0 S/cm to 0.0548 S/cm (preferably 1.0 $\mu$S/cm to 0.1 pS/cm). More specifically, ion exchange water, reverse osmosis water, or distilled water, which each exhibit the above conductivity are used. Preferably, ion exchange water is used. Exemplary methods for producing deionized water having such conductivity encompass a method in which (i) water such as tap water is subjected as necessary to filtration with use of a filter or activated carbon, (ii) the water is subjected to decarboxylation or degassing, and thereafter (iii) the water is subjected to treatment with an ion-exchange membrane or a reverse osmosis membrane, or subjected to distillation.

The tank 420 stores therein the liquid 423. The liquid 423 is introduced into the cell 411 through an L-shaped tube 422 having a cock. A glass tube 421 is inserted into the tank 420, and the inside of the glass tube 421 is filled with air. This makes it possible to make the height of the lower end of the glass tube 421 and the liquid level in the cell 411 the same. That is, while the level of the liquid 423 in the tank 420 is higher than the lower end of the glass tube 421, the liquid level in the cell 411 can be kept constant. In this measurement, the difference in height between the lower level of the liquid 423 in the tank 420 (i.e., the lower end of the glass tube 421) and the bottom surface of the swollen gel 414 is 4 cm.

In this manner, the device 400 makes it possible to introduce the liquid 423 having a constant hydrostatic pressure into the cell 411. Because the piston 412 has holes 415, the liquid 423 flows through the holes 415, through a layer of the swollen gel 414, and then out of the cell 411. The container 410 rests on a stainless steel metal gauze 431 that does not obstruct the passage of the liquid 423. The liquid 422 flowing out of the cell 411 is therefore ultimately collected in a collection container 432. The amount of the liquid 422 collected in the collection container 432 can be weighed by a scale balance 433.

A specific method of measuring the gel passage rate (GPR) is as follows. The following operations are carried out at room temperature (20°C to 25°C).

(1) The particulate water-absorbing agent (0.900 g) is placed in the cell 411 in a uniform manner.
(2) The particulate water-absorbing agent is made to absorb liquid (0.90 mass% aqueous sodium chloride solution) for 60 minutes without load, so as to obtain the swollen gel 414.
(3) A piston is placed on the swollen gel 414, and a pressure of 0.3 psi (2.07 kPa) is applied.
(4) Liquid 423 is introduced into the cell 411 and allowed to flow through the swollen gel 414 layer, while a constant hydrostatic pressure of 3923 dyne/cm$^2$ is maintained.
(5) The amount of liquid 423 that passes through the swollen gel 414 layer is recorded at 5 second intervals for 3 minutes to measure the flow rate of the liquid 423 passing through the swollen gel 414 layer. Measurements are carried out using a scale balance 433 and a computer (not shown).
(6) The flow rate in the period from 1 minute after the start of the flow of the liquid 423 to 3 minutes after the start of the flow of the liquid 423 is averaged so as to obtain the gel permeation rate (GPR) [g/min].

(Method of Adjusting Gel Permeation Rate (GPR))

The gel permeation rate (GPR) can be adjusted by, for example: adjusting a particle size distribution by a pulverizing step and a classification step in the method of producing the particulate water-absorbing agent described later (for example, a larger average particle diameter tends to correlate to a higher GPR value); a surface-crosslinking step (for example, the GPR can be adjusted by adjusting e.g. the amount of the surface-crosslinking agent); and an other additive adding step (for example, adding an additive that improves GPR). Specifically, in order to reduce the gel permeation rate (GPR), the average particle diameter of the particulate water-absorbing agent can be reduced, and/or the added amount of the surface-crosslinking agent can be reduced. Conversely, in order to increase the gel permeation rate (GPR), the average particle diameter of the particulate water-absorbing agent can be increased, and/or the added amount of the surface-crosslinking agent can be increased. A particulate water-absorbing agent having a desired gel permeation rate may also be obtained by mixing two or more types of particulate water-absorbing agent having different gel permeation rates (GPRs).

[4-2. Surface Tension]

Surface tension indicates, on a per-unit-area basis, the work (free energy) required to increase the surface area of a solid or a liquid. The surface tension of the particulate water-absorbing agent (the first particulate water-absorbing agent and the second particulate water-absorbing agent) contained in a water-absorbing sheet in accordance with an embodiment of the present invention refers to the surface tension of a liquid (0.90 mass% aqueous sodium chloride solution) when the particulate water-absorbing agent or the water-absorbing resin has been dispersed in that liquid. A specific method of measuring the surface tension in the Examples of the present application is as follows.

First, 50 mL of a liquid (0.90 mass% aqueous sodium chloride solution) adjusted to 20°C is placed in a sufficiently washed 100 mL beaker, and the surface tension is measured using a surface tension meter (K11 automatic surface tension meter, manufactured by KRUSS).
It is confirmed that the surface tension value in this measurement is 71 to 75 [mN/m], to ensure the accuracy of the measurement.

Next, a sufficiently washed fluorocarbon resin rotor having a length of 25 mm is placed into the beaker containing the liquid (20°C, after surface tension measurement), 0.5 g of the particulate water-absorbing agent or the water-absorbing resin are added, and stirring is performed at 500 rpm for 4 minutes. After 4 minutes, the stirring is stopped, and after the particulate water-absorbing agent or the water-absorbing resin containing water has settled, the surface tension of a supernatant liquid is measured. In the Examples described later, a plate method using a platinum plate was employed. Prior to each measurement, the platinum plate is sufficiently washed with deionized water and also further washed while being heated with use of a gas burner, before being used for measurement.

In order to bring about an effect of the present invention, (i.e., to provide a water-absorbing sheet which, with respect to urine having a wide range of salt concentrations (mass%) including 0, has a favorable liquid absorption speed and favorable re-wet, and which has good comfortability for users, including infants, whose urine tends to change in salt concentration due to a change in the user's physical condition), the surface tensions of the first particulate water-absorbing agent and the second particulate water-absorbing agent are preferably as follows.

The surface tension of the first particulate water-absorbing agent and/or the second particulate water-absorbing agent is preferably 65 mN/m or more, more preferably 66 mN/m or more, even more preferably 67 mN/m or more, even more preferably 69 mN/m or more, even more preferably 70 mN/m or more, even more preferably 71 mN/m or more, and even more preferably 72 mN/m or more. Because the water-absorbing sheet is more prone the effects of the surface tension of the particulate water-absorbing agent than are conventional disposable diapers, the value of the surface tension is particularly important.

[4-3. Particle Shape (Non-Uniformly Pulverized Shape)]

The first particulate water-absorbing agent and/or the second particulate water-absorbing agent preferably contain a particulate water-absorbing agent having a non-uniformly pulverized shape. Particles having a non-uniformly pulverized shape have a larger specific surface area than spherical particles (obtained by reversed phase suspension polymerization or vapor phase polymerization) and therefore have a high liquid absorption speed and are more easily fixed to a sheet.

The term "non-uniformly pulverized shape" means that the particles are pulverized so as to have an uneven shape. Herein, the term "non-uniformly pulverized shape" indicates the shape of a crushed substance obtained by crushing a hydrogel of a crosslinked polymer during or after polymerization (or by crushing a dried material of such a hydrogel). Spherical particles or a granulated material of spherical particles, obtained by a method of producing a water-absorbing resin without a pulverizing step (typically, e.g. reversed phase suspension polymerization or droplet polymerization) do not have the non-uniformly pulverized shape.

In order to bring about an effect of the present invention, (i.e., to provide a water-absorbing sheet which, with respect to urine having a wide range of salt concentrations (mass%) including 0, has a favorable liquid absorption speed and favorable re-wet, and which has good comfortability for users, including infants, whose urine tends to change in salt concentration due to a change in the user's physical condition), the proportion of a particulate water-absorbing agent having the non-uniformly pulverized shape in the first particulate water-absorbing agent, and the proportion of the particulate water-absorbing agent having the non-uniformly pulverized shape in the second particulate water-absorbing agent are preferably as follows.

The proportion of the particulate water-absorbing agent having the non-uniformly pulverized shape in the first particulate water-absorbing agent is preferably 50 mass% or more, more preferably 60 mass%, even more preferably 70 mass%, even more preferably 80 mass%, even more preferably 90 mass%, and even more preferably 100 mass%, with respect to the total amount of each particulate water-absorbing agent in the first particulate water-absorbing agent. Similar ranges are suitable for the proportion of the particulate water-absorbing agent having the non-uniformly pulverized shape in the second particulate water-absorbing agent.

As a particulate water-absorbing agent for use in the water-absorbing sheet in accordance with an embodiment of the present invention, it is preferable to use particles having the non-uniformly pulverized shape which particles are obtained by pulverizing dried material of a crosslinked polymer hydrogel. Similarly, as a particulate water-absorbing agent for use in a water-absorbing sheet in accordance with an embodiment of the present invention, it is preferable to use particles having the non-uniformly pulverized shape which particle are obtained by pulverizing a crosslinked polymer obtained by aqueous solution polymerization.

[4-4. PSD (ERT 420.2-02)]

The term "PSD" is an acronym for "particle size distribution", and means a particle size distribution of a particulate water-absorbing agent or a water-absorbing resin, as measured by sieve classification.

In relation to this, a mass average particle diameter (D50) and a logarithmic standard deviation ($\sigma\zeta$) of a particle size distribution are measured according to methods similar to those disclosed in the Specification of US Patent No. 7638570 (see "(3) Mass-Average Particle Diameter (D50) and Logarithmic Standard Deviation ($\sigma\zeta$) of Particle Diameter Distribution" in US Patent No. 7638570).

The particle size distribution (PSD) of a particulate water-absorbing agent and the logarithmic standard deviation ($\sigma\zeta$) of a particle size distribution are measured in accordance with the measurement methods disclosed in U.S. Patent Application Publication No. 2006/204755.

Specifically, 10.00 g of the particulate water-absorbing agent is classified by using JIS standard sieves (The IIDA TESTING SIEVE: 80 mm in inner diameter; JIS Z8801-1 (2000)) having respective mesh sizes of 850 μm, 710 μm, 600 μm, 500 μm, 425 μm, 300 μm, 212 μm, 150 μm, 106 μm, and 75 μm, or sieves corresponding to the JIS standard sieves. After this classification, the mass of each sieve is measured, and the mass percentage (mass%) of particles having a particle diameter of less than 150 μm is calculated. The "mass percentage of particles having a particle diameter of less than 150 μm" refers to the proportion (%), by mass, of particles which can pass through a JIS standard sieve having a mesh size of 150 μm, relative to the entire amount of the particulate water-absorbing agent.

As for the mass average particle diameter (D50), a graph of a residual percentage R of each particle size mentioned above is plotted on a logarithmic probability paper, and a particle diameter corresponding to R = 50 mass% is then read as the mass average particle diameter (D50) from the graph. In other words, the mass average particle diameter (D50) is a particle diameter corresponding to 50 mass% in the particle size distribution of the entire amount of the particulate water-absorbing agent.

The logarithmic standard deviation (σζ) of the particle size distribution is expressed by the following Formula (1). A smaller value of σζ indicates a narrower particle size distribution.

$$\sigma\zeta = 0.5 \times \ln (X2/X1) \qquad \ldots \text{Formula (1)}$$

where X1 is the particle diameter when R = 84.1 mass%, and X2 is the particle diameter when R = 15.9 mass%.

[4-4-1. Particle Size (Mass Average Particle Diameter (D50), Particle Size Distribution, and Logarithmic Standard Deviation (σζ) of Particle Size Distribution)]

The mass average particle diameter (D50) of the first particulate water-absorbing agent and/or mass average particle diameter (D50) of the second particulate water-absorbing agent is preferably 200 μm to 600 μm, more preferably 200 μm to 550 μm, even more preferably 250 μm to 500 μm, and even more preferably 350 μm to 450 μm.

The proportion of particles having a particle diameter of less than 150 μm in the first particulate water-absorbing agent and/or the proportion of particles having a particle diameter of less than 150 in the second particulate water-absorbing agent is preferably 10 mass% or less, more preferably 5 mass% or less, and even more preferably 1 mass% or less. The proportion of particles having a particle diameter of 850 μm or more in the first particulate water-absorbing agent and/or the proportion of particles having a particle diameter of 850 μm or more in the second particulate water-absorbing agent is preferably 5 mass% or less, more preferably 3 mass% or less, and even more preferably 1 mass% or less. A lower limit value of each of the proportions of such particles is preferably as low as possible and is desirably 0 mass%. However, there is no particular problem even if the lower limit value is approximately 0.1 mass%.

The logarithmic standard deviation (σζ) of the particle size distribution of the first particulate water-absorbing agent and/or logarithmic standard deviation (σζ) of the particle size distribution of the second particulate water-absorbing agent is preferably 0.20 to 0.50, more preferably 0.25 to 0.40, and even more preferably 0.27 to 0.35.

The properties relating to particle size described above can be measured in accordance with the measurement methods disclosed in e.g. US Patent No. 7638570 or EDANA ERT420.2-02, with use of standard sieves.

[4-5. Fluid Retention Capacity Without Load After Suspension (FSCw)]

FSCw (fluid retention capacity without load after suspension) is measured in accordance with ERT 440.2-02, under the following conditions.

(1) First, 0.200 g of the particulate water-absorbing agent is weighed out and placed uniformly into a nonwoven fabric bag (60 mm × 85 mm). The bag is then heat-sealed. Thereafter, the bag is immersed in 500 mL of deionized water that has been adjusted to have a temperature of 23±1°C.

(2) After 20 seconds, the bag is pulled up and drained by suspending the bag for 1 minute. Thereafter, the mass W3 [g] of the bag is measured.

(3) Procedures similar to (1) and (2) are carried out, except that no particulate water-absorbing agent is put into the nonwoven fabric bag. The mass W4 [g] of that bag is measured. Using the values of W3 and W4, the FSCw (fluid retention capacity without pressure) is calculated in accordance with Formula (2) below.

FSCw (g/g) = {(W3 - W4)/(mass of particulate water-absorbing agent (0.200 g))} - 1       Formula (2)

[4-6. Centrifuge Retention Capacity (CRC)]

The term "CRC" is an abbreviation for "centrifuge retention capacity" (synonymous with absorption capacity without load). The CRC can be measured in accordance with an EDANA method (ERT 441.2-02).

[5. Method of Producing Particulate Water-Absorbing Agent]

The following description will discuss an example of a method of producing a particulate water-absorbing agent which can be used for a water-absorbing sheet in accordance with an embodiment of the present invention.

[5-1. Aqueous Monomer Solution Preparation Step]

This step is a step of preparing an aqueous solution containing a monomer (e.g., acrylic acid (salt)) as a main component (this aqueous solution hereinafter referred to as an "aqueous monomer solution"). Note that a monomer slurry liquid may be used instead as long as the water-absorbing resin obtained does not have degraded water absorption performance. For convenience of description, however, an aqueous monomer solution is described here.
The wording "contains as a main component" means that amount of the acrylic acid (salt) used when polymerizing polyacrylic acid (salt) is ordinarily 50 mol% to 100 mol%, preferably 70 mol% to 100 mol%, more preferably 90 mol% to 100 mol%, and even more preferably substantially 100 mol%, relative to a total amount of monomers for use in polymerization (excluding an internal crosslinking agent).

(Acrylic Acid and Acrylic Acid Salt)

In this step, it is preferable that an acrylic acid and/or a salt thereof (hereinafter referred to as "acrylic acid (salt)") is used as a monomer, from the viewpoint of physical properties of the particulate water-absorbing agent to be produced, and from the viewpoint of productivity.
The acrylic acid salt is obtained by neutralizing the acrylic acid with use of a basic composition. The acrylic acid salt may be a commercially available acrylic acid salt (such as sodium acrylate) or may be obtained in the production process of the particulate water-absorbing agent.
The "basic composition" in this step refers to a composition containing a basic compound. Examples of the basic compound encompass: a carbonate, bicarbonate, or hydroxide of an alkali metal; ammonia; and organic amine. Of these, a basic composition having strong basicity is preferable from the viewpoint of the physical properties of the particulate water-absorbing agent to be obtained. As such, the basic compound is preferably a hydroxide of alkali metal (such as sodium hydroxide, potassium hydroxide, or lithium hydroxide), and is more preferably sodium hydroxide.
In order to neutralize acrylic acid to obtain acrylic acid (salt), acrylic acid may be neutralized prior to polymerization, or a crosslinked hydrogel polymer obtained by crosslinking and polymerizing acrylic acid may be neutralized (hereinafter, the latter is referred to as "later neutralization"). Neutralization before polymerization and later neutralization may be used in combination. The step of neutralization is not limited to any particular type, and may be of a continuous type or a batch type. However, the continuous type is preferable from the viewpoint of efficiency.
Note that with regard to conditions such as the apparatus used for neutralization, a neutralization temperature, and a retention time, the conditions disclosed in International Publication No. WO 2009/ 123197 and U.S. Patent Application Publication No. 2008/0194863 can be applied.
The neutralization rate of the acrylic acid (salt) is preferably 10 mol% to 90 mol%, more preferably 40 mol% to 85 mol%, even more preferably 50 mol% to 80 mol%, and particularly preferably 60 mol% to 75 mol%, relative to acid groups of a monomer. A neutralization rate that is less than 10 mol% may result in a significant decrease in fluid retention capacity. With a neutralization rate that is higher than 90 mol%, it may not be possible to obtain a water-absorbing resin having a high fluid retention capacity under pressure.

(Other Monomer(s))

The "other monomer(s)" in this step refer to monomers other than the acrylic acid (salt). The particulate water-absorbing agent can be produce with use of acrylic acid (salt) and other monomer(s) in combination.
Examples of the other monomer(s) encompass an unsaturated monomer which is water-soluble or hydrophobic. As a more specific example, the compounds disclosed in U.S. Patent Application Publication No. 2005/0215734 (excluding acrylic acid) may be used.

(Internal Crosslinking Agent)

For an internal crosslinking agent used in the production of the particulate water-absorbing agent, the compounds disclosed in US Patent No. 6241928 may be used. From these compounds, one or more kinds of compounds are selected in consideration of reactivity.

The amount of internal crosslinking agent used is preferably 0.0001 mol% to 10 mol%, and more preferably 0.001 mol% to 1 mol%, relative to the total amount of monomers. Setting the used amount to fall within the above range makes it possible to obtain a desired water-absorbing resin. If the used amount is too small, there tends to be a decrease in gel strength and an increase in a water-soluble component, which is not preferable. Conversely, if the used amount is too great, there tends to be a decrease in the fluid retention capacity, which is not preferable. GPR can be adjusted by adjusting the used amount of the internal crosslinking agent. Specifically, if the used amount of the internal crosslinking agent is increased, the GPR tends to increase. Conversely, if the used amount of the internal crosslinking agent is decreased, the GPR tends to decrease.

The method of using the internal crosslinking agent is preferably a method in which a certain amount of the internal crosslinking agent is added in advance to an aqueous monomer solution, and then polymerization and a crosslinking reaction are carried out simultaneously. Other example methods that may be employed encompass the following: (1) a method in which an internal crosslinking agent is added during or after the polymerization so that post-crosslinking is carried out; (2) a method in which radical crosslinking is carried out with use of a radical polymerization initiator; and (3) a method in which radiation crosslinking is carried out with use of active energy rays such as an electron ray or an ultraviolet ray. These methods may be used in combination.

(Other Substances Added to Aqueous Monomer Solution)

In this step, other substances may be added in the step of preparing the aqueous monomer solution, in order to improve the physical properties of the resulting water-absorbing resin.

Examples of other substances encompass hydrophilic polymers (such as starch, a starch derivative, cellulose, a cellulose derivative, polyvinyl alcohol, polyacrylic acid (salt), and crosslinked polyacrylic acid (salt)).

If a water-soluble resin or a water-absorbing resin is used as the hydrophilic polymer, a graft polymer or a water-absorbing resin composition (for example, a polymer produced from starch and an acrylic acid or a polymer produced from PVA and an acrylic acid) can be obtained. Such a graft polymer or water-absorbing resin composition may be used in the water-absorbing sheet in accordance with an embodiment of the present invention.

(Monomer Component Concentration)

In this step, the aqueous monomer solution is prepared by adding each of the above mentioned substances to water. The concentration of the monomer component in the aqueous monomer solution is not particularly limited. However, considering the physical properties of the obtained water-absorbing resin, the concentration of the monomer component is preferably 10 mass% to 80 mass%, more preferably 20 mass% to 75 mass%, and even more preferably 30 mass% to 70 mass%.

The "concentration of the monomer component" is a value obtained by Formula (3) below. Note that the "mass of the aqueous monomer solution" in the formula does not include the mass of the graft component, the mass of the water-absorbing resin, or the mass of a solvent in reversed phase suspension polymerization other than water.

Monomer component concentration [mass%] = (mass of monomer component / mass of aqueous monomer solution) $\times$ 100          Formula (3).

If the method of polymerization employed is aqueous solution polymerization or reversed phase suspension polymerization, solvents other than water can be used in combination. The type of the solvent used is not limited to any particular one.

[5-2. Polymerization Step]

This step is a step of polymerizing an acrylic acid (salt)-based aqueous monomer solution obtained in the step of preparing the aqueous monomer solution, so that a crosslinked hydrogel polymer (hereinafter referred to as "hydrogel") is obtained.

(Polymerization Initiator)

A polymerization initiator used in this step is selected as appropriate in accordance with e.g. the polymerization method, and is not particularly limited. Examples of the polymerization initiator encompass a pyrolytic-type polymerization initiator, a photolytic-type polymerization initiator, and a redox-type polymerization initiator (a polymerization initiator in combination with a reducing agent, which facilitates decomposition of a pyrolysis-type polymerization initiator and/or a photolytic-type polymerization initiator). Specifically, one or more of the polymerization initiators disclosed in US Patent No. 7265190 can be used as the polymerization initiator. From the viewpoint of the handleability of the polymerization initiator and the physical properties of the obtained particulate water-absorbing agent or water-absorbing resin, the polymerization initiator is preferably a peroxide or an azo compound, more preferably a peroxide, and even more preferably a persulfate.

(Form of Polymerization)

The polymerization method used in this step can be droplet polymerization, aqueous solution polymerization, or reversed phase suspension polymerization. Aqueous solution polymerization or reversed phase suspension polymerization are preferable, and aqueous solution polymerization is more preferable. For aqueous solution polymerization, continuous aqueous solution polymerization is preferable. The continuous aqueous solution polymerization may be continuous belt polymerization or continuous kneader polymerization.

[5-3. Gel-Crushing Step]

This step is a step of gel-crushing a hydrogel, which has been obtained by the polymerization step, with use of, for example, a screw extruder (such as a kneader or a meat chopper), or a gel-crusher (such as a cutter mill), in order to obtain a hydrogel in the form of particles (hereinafter referred to as "particulate hydrogel"). If kneader polymerization is employed in the polymerization step, the polymerization step and the gel-crushing step proceed simultaneously. The gel-crushing step may be omitted if a polymerization method is employed in which the particulate hydrogel is obtained directly in the process of polymerization (such as vapor phase polymerization or reversed phase suspension polymerization).

[5-3-1. Moisture Content]

The moisture content of the hydrogel is 50 mass% or more, and preferably 52 mass% or more. Setting the moisture content to fall within the above range makes it possible to obtain a particulate water-absorbing agent having excellent physical properties. The moisture content is measured in accordance with an EDANA method (ERT 430.2-02). Note that in the Examples described later, in measurements of the moisture content, the amount of the sample was changed to 1.0 g, the drying temperature was changed to 180°C, and the drying time was changed to 24 hours.

[5-4. Drying Step]

This step is a step of drying the particulate hydrogel, which has been obtained by the polymerization step and/or the gel-crushing step, until a desired resin solid content is attained, so as to obtain a dried polymer. The resin solid content is calculated from drying loss of the water-absorbing resin (i.e., a change in mass after heating 1 g of the water-absorbing resin at 180°C for three hours). The dry solid content of the water-absorbing resin (particulate water-absorbing agent) is preferably 80 mass% or more, more preferably 85 mass% to 99 mass%, even more preferably 90 mass% to 98 mass%, and particularly preferably 92 mass% to 97 mass%.

The method of drying the particulate hydrogel is not particularly limited. For example, the following methods can be employed: thermal drying, hot air drying, drying under reduced pressure, fluidized bed drying, infrared drying, microwave drying, drum dryer drying, drying by azeotropic dehydration with a hydrophobic organic solvent, and high humidity drying by use of high temperature water vapor. Out of these examples, from the viewpoint of drying efficiency, the drying method is preferably hot air drying, and more preferably band drying (hot air drying which is performed on a through-flow belt).

If band drying is employed, the various conditions disclosed in, for example, International Publication Nos. WO 2006/100300, WO 2011/025012, WO 2011/025013, and WO 2011/111657 can be applied as appropriate.

[5-5. Pulverizing Step and Classification Step]

The pulverizing step is a step of pulverizing the dried polymer obtained in the drying step. The classification step is a step of adjusting the pulverized polymer obtained in the pulverizing step to have a particle size in a predetermined range, so as to obtain a water-absorbing resin powder. Herein, the water-absorbing resin in powder form prior to surface-crosslinking is referred to as a "water-absorbing resin powder" for convenience.

Examples of apparatuses used in the pulverizing step encompass a high-speed rotation pulverizer (such as a roll mill, a hammer mill, a screw mill, or a pin mill), a vibration mill, a knuckle-type pulverizer, and a cylindrical mixer. These apparatuses may be used in combination as necessary.

The method of particle size adjustment used in the classification step is not particularly limited. Example methods encompass sieve classification with use of a JIS-standard sieve (JIS Z 8801-1(2000)) and airflow classification.

Note that the adjustment of the particle size of the water-absorbing resin is not limited to being carried out during the pulverizing step and classification step, but may be carried out during the polymerization step (in particular, if reversed phase suspension polymerization or droplet polymerization are employed) or other steps (for example, a granulation step or fine powder recovery step).

The water-absorbing resin powder obtained in this step is preferably adjusted to have the particle size of the particulate water-absorbing agent described in section [4-4]. GPR can be adjusted by adjusting the particle size of the water-absorbing resin powder. Specifically, if the average particle diameter of the water-absorbing resin powder is increased, GPR tends to increase. Conversely, if the average particle diameter of the water-absorbing resin powder is decreased, GPR tends to decrease.

The particle size described as being preferable in [4-4] is also preferable for the water-absorbing resin powder prior to surface-crosslinking and for the water-absorbing resin after surface crosslinking (hereinafter also referred to as "water-absorbing resin particles" for convenience). As such, it is preferable to carry out the surface-crosslinking step such that the water-absorbing resin particles after crosslinking have the above particle size. Further, it is more preferable to provide a sizing step after the surface-crosslinking step to re-adjust the particle size.

[5-6. Surface-Crosslinking Step]

This step is a step of forming, in a surface layer of the water-absorbing resin powder produced through the above steps (that is, in a portion of the water-absorbing resin powder which portion is up to several tens of micrometers deep from the surface), a portion with a higher crosslinking density. The surface-crosslinking step includes a mixing step, a heat treatment step, and optionally a cooling step.

In this step, the surface of the water-absorbing resin powder is surface-crosslinked by, for example, radical crosslinking, surface polymerization, or a crosslinking reaction with the surface-crosslinking agent. This produces a surface-crosslinked water-absorbing resin (the water-absorbing resin particles).

(Surface-Crosslinking Agent)

The surface-crosslinking agent used in this step is not particularly limited. Examples of the surface-crosslinking agent encompass organic surface-crosslinking agents and inorganic surface-crosslinking agents. An organic surface-crosslinking agent that is reactive with a carboxyl group is preferable, from the viewpoint of the physical properties of the water-absorbing resin to be obtained and the handleability of the surface-crosslinking agent. Such a crosslinking agent may be, for example, one or more of the surface-crosslinking agents disclosed in U.S. Patent 7183456. More specific examples of the surface-crosslinking agent encompass a polyhydric alcohol compound, an epoxy compound, a haloepoxy compound, a polyamine compound, a condensate of a polyamine compound and a haloepoxy compound, an oxazoline compound, an oxazolidinone compound, a polyvalent metal salt, an alkylene carbonate compound, and a cyclic urea compound. The GPR can be adjusted by adjusting the added amount of the surface-crosslinking agent. Specifically, if the added amount of the surface-crosslinking agent is increased, the GPR tends to increase. Conversely, if the added amount of the surface-crosslinking agent is decreased, the GPR tend to decrease.

(Mixing Step)

This step is a step of mixing the water-absorbing resin powder and the surface-crosslinking agent. The method of mixing the surface-crosslinking agent is not particularly limited. One possible example is a method in which a surface-crosslinking agent solution is prepared in advance and the solution is added to the water-absorbing resin powder. In such a method, the surface-crosslinking agent solution is preferably sprayed or dropped on the water-absorbing resin powder, and more preferably sprayed.

The device used in the mixing step is not particularly limited. The device is preferably a high-speed stirring mixer, and more preferably a high-speed stirring continuous mixer.

(Heat Treatment Step)

This step is a step of heating a mixture, which has been obtained in the mixing step, so as to cause a crosslinking reaction on the surface of the water-absorbing resin powder.

The device used for heating the mixture is not particularly limited. Preferably, a paddle dryer is used.

(Cooling Step)

This step is an optional step which is carried out as necessary after the heat treatment step.

The device used for cooling is not particularly limited. The device used for cooling is preferably the same device as that used in the heat treatment step, and is more preferably a paddle dryer.

[5-7. Remoistening Step (Step of Adding Additives)

The remoistening step is a step of adding a small amount of water (approximately 0.1 mass% to 20 mass%, where the mass of the water-absorbing resin particles is 100 mass%) to the water-absorbing resin particles after surface-crosslinking. In this step, at least one additive selected from the group consisting of polyvalent metal salt compounds, polycationic polymers, chelating agents, inorganic reducing agents, hydroxycarboxylic acid compounds, and moisture absorption fluidity improving agents may be added as necessary to the water-absorbing resin particles obtained in the surface-crosslinking step.

[5-8. Step of Adding Other Additives]

In the method of producing the particulate water-absorbing agent described in this section, additives other than the additives described above may be added to impart various functions to the water-absorbing resin. Examples of such additives encompass a surfactant, a compound having a phosphorus atom, an oxidizer, an organic reducing agent, water-insoluble inorganic fine particles, organic powder (such as metallic soap), a deodorant, an antimicrobial agent, pulp, and thermoplastic fibers. Adding such additives can increase the GPR in some cases. As such, it is also possible to adjust the GPR in this step.

[5-9. Other Steps]

The method of producing the particulate water-absorbing agent described in this section may include as necessary e.g. a granulation step, a sizing step, a fine powder removal step, and a fine powder recycling step, in addition to the steps described above. Moreover, it is possible to further carry out one or more steps such as a transportation step, a storing step, a packing step, and a reserving step.

Of the example steps described above, the "sizing step" encompasses a fine powder removal step carried out after the surface-crosslinking step, and a step of carrying out classification and pulverization in a case where the water-absorbing resin has aggregated so as to have a size larger than the desired size. Further, the "fine powder recycling step" includes (i) a step of adding fine powder as is to any step of the production process of the water-absorbing resin, and (ii) a step of adding fine powder, in the form of a large hydrogel, to any step of the production process of the water-absorbing resin.

[Configurations of the Present Invention]

In a first aspect, the present invention as defined in claim 1 is directed to a water-absorbing sheet including:

a first sheet; a second sheet; and
a particulate water-absorbing agent sandwiched between the first sheet and the second sheet,
at least one sheet selected from the first sheet and the second sheet being a water-permeable sheet,
the particulate water-absorbing agent including: (1) a first particulate water-absorbing agent localized in the vicinity of the first sheet; and (2) a second particulate water-absorbing agent localized in the vicinity of the second sheet,
the first particulate water-absorbing agent having a gel permeation rate (GPR) which is higher than that of the second particulate water-absorbing agent,
the gel permeation rate (GPR) of the first particulate water-absorbing agent differing by 10 g/min or more from that of the second particulate water-absorbing agent,
the gel permeation rate (GPR) of the second particulate water-absorbing agent being 50 g/min or less. Preferred embodiments are subject of the dependent claims.

In a second aspect, the present invention as defined in claim 9 relates to method of producing the water-absorbing sheet according to the first aspect, including at least one of the following steps:

    (1) dispersing a first particulate water-absorbing agent onto a first sheet;
    (2) dispersing a second particulate water-absorbing agent onto a second sheet; and
    (3) dispersing the first particulate water-absorbing agent and/or the second particulate water-absorbing agent onto an intermediate sheet,

        at least one sheet selected from the first sheet and the second sheet being a water-permeable sheet,
        the first particulate water-absorbing agent having a gel permeation rate (GPR) which is higher than that of the second particulate water-absorbing agent,
        the gel permeation rate (GPR) of the first particulate water-absorbing agent differing by 10 g/min or more from that of the second particulate water-absorbing agent,
        the gel permeation rate (GPR) of the second particulate water-absorbing agent being 50 g/min or less.

In a third aspect, the present invention as defined in claim 10 is directed to an absorbent article including:

    a liquid-permeable sheet;
    a liquid-impermeable sheet; and
    the water-absorbing sheet according to the first aspect of the present invention, which is sandwiched between the liquid-permeable sheet and the liquid-impermeable sheet.

Examples

The following description will discuss the present invention in more detail with use of Production Examples, Examples, and Comparative Examples. However, it will be understood that the present invention is not limited to these Production Examples, Examples, and Comparative Examples. The present invention also encompasses examples derived by combining technical means disclosed in the various Examples.

Electric devices used in the Production Examples, Examples, and Comparative Examples (including electric devices used to measure physical properties of a particulate water-absorbing agent) each used a 200-V or 100-V electric power supply, unless otherwise specified. The physical properties of particulate water-absorbing agents were measured at room temperature (20°C to 25°C) and at a relative humidity of 50% RH, unless otherwise specified.

[Physical Property Measurements for the Particulate Water-Absorbing Agent]

Methods of measuring the physical properties of the particulate water-absorbing agent were as described above in sections [4. Physical Properties of Particulate Water-Absorbing Agent] and [5. Method of Producing Particulate Water-Absorbing Agent]. If the object subjected to measurement is something other than the particulate water-absorbing agent (for example, a particulate hydrogel), the wording "particulate water-absorbing agent" in the descriptions below can be replaced with "particulate hydrogel".

[Method of Evaluating Water-Absorbing Sheet (Liquid Absorption Speed and Re-wet)]

The following description will discuss a method of evaluating the water-absorbing sheet in the present application.

(Flat State Evaluation of Water-Absorbing Sheet)

Using the container shown in Fig. 3 (inner dimensions: 8 cm in width, 16 cm in length, 3.5 cm in height; made of plastic), the water-absorbing sheet was evaluated in a flat state.

First, a water-absorbing sheet was cut to have a size of 8 cm in width and 16 cm in length, and was placed in the container so that the bottom surface of the water-absorbing sheet matched the bottom of the container, with substantially no gaps. In this state, 250 mL of deionized water at 23°C was quickly introduced. A time period from when the liquid was introduced to when the liquid disappeared from the surface of the water-absorbing sheet was considered to be a flat-state surface liquid absorption speed (seconds).

Three minutes after the liquid was introduced, 30 sheets of filter paper whose mass had been measured in advance (model no. 2, manufactured by ADVANTEC; cut to a size of 7.9 cm in width $\times$ 15.9 cm in length) were placed on the water-absorbing sheet, and a weight (6514 g) having the same size as the filter paper (7.9 cm in width $\times$ 15.9 cm in length) was placed on the filter paper for 10 seconds. After 10 seconds, the weight was removed, and flat-state re-wet (g) was measured from the increase in the mass of the filter paper.

If the flat-state surface liquid absorption speed is 50 seconds or less, it can be said that the absorption speed is sufficiently high. The flat-state surface liquid absorption speed is preferably 48 seconds or less, and more preferably 46 seconds or less. If the flat-state re-wet is 1.8 g or less, it can be said that the re-wet is sufficiently small. The flat-state re-wet is preferably 1.5 g or less, and more preferably 1.2 g or less. Further, from the viewpoint of the balance between the flat-state surface liquid absorption speed and the re-wet, it is preferable to achieve both a flat-state surface liquid absorption speed of 48 seconds or less and a flat-state re-wet of 1.8 g or less.

The following description will discuss Production Examples of the particulate water-absorbing agent. Note, however, that these are merely examples, and that the particulate water-absorbing agent can be produced by an appropriate combination of known techniques.

[Production Example 1]

First, there was prepared an aqueous monomer solution (1) consisting of 300 parts by mass of acrylic acid, 100 parts by mass of a 48 mass% aqueous sodium hydroxide solution, 0.94 parts by mass of polyethylene glycol diacrylate (average n number: 9), 16.4 parts by mass of a 0.1 mass% aqueous trisodium diethylenetriamine pentaacetate solution, and 314.3 parts by mass of deionized water.

Next, 150.6 parts by mass of a 48 mass% aqueous sodium hydroxide solution was mixed with the aqueous monomer solution (1), whose temperature had been adjusted to 38°C.

Next, 14.6 parts by mass of a 4 mass% aqueous sodium persulfate solution was added. Thereafter, the aqueous monomer solution (1) was introduced into a planar polymerization device in a manner so as to have a thickness of 10 mm. Thereafter, polymerization was allowed to proceed (polymerization time: 3 minutes), so that a hydrogel (1) was obtained.

The hydrogel (1) thus obtained had a moisture content of 50.5 mass%.

The hydrogel (1) thus obtained was gel-crushed with use of a screw extruder, so that a particulate hydrogel (1) was obtained.

The particulate hydrogel (1) obtained after the gel-crushing had a moisture content of 50.9 mass%, a mass average particle diameter (D50) of 994 $\mu$m, and a logarithmic standard deviation ($\sigma\zeta$) of a particle size distribution of 1.01.

Within one minute after completion of the gel-crushing, the particulate hydrogel (1) was subjected to drying by flow therethrough of hot air at 185°C for 30 minutes, with use of a through-flow dryer, so that a dried polymer (1) was obtained.

The dried polymer (1) obtained after drying was pulverized and then classified with use of JIS standard sieves having respective mesh sizes of 710 $\mu$m and 175 $\mu$m, so that a water-absorbing resin powder (1) having a non-uniformly pulverized shape was obtained. The water-absorbing resin powder (1) had a mass average particle diameter (D50) of 348 $\mu$m, a logarithmic standard deviation ($\sigma\zeta$) of a particle size distribution of 0.32, a CRC of 42.1 g/g, and a proportion of particles having a particle diameter of less than 150 $\mu$m (a proportion of particles that can pass through a sieve having a mesh size of 150 $\mu$m) of 0.5 mass%.

To 100 parts by mass of the water-absorbing resin powder (1), a surface-crosslinking agent solution (1) consisting of 0.03 parts by mass of ethylene glycol diglycidyl ether, 0.4 parts by mass of 1,4-butanediol, 0.6 parts by mass of propylene glycol, and 3.0 parts by mass of deionized water was added. Then, a resultant mixture was mixed until uniform. Thereafter, the mixture was heat-treated at 190°C for approximately 45 minutes so that resulting water-absorbing resin particles (1) would have a CRC of 33 g/g. Then, the mixture was force-cooled to 60°C.

Next, the water-absorbing resin particles (1) obtained via the above operations were subjected to a paint shaker test so as to be subjected to damage equivalent to that occurring in a production process. Thereafter, to 100 parts by mass of the water-absorbing resin powder (1), 1.01 parts by mass of an aqueous chelating agent solution (1) consisting of 0.01 parts by mass of trisodium diethylenetriamine pentaacetate and 1 part by mass of deionized water was added. Then, a resultant mixture was mixed until uniform. Thereafter, the mixture was dried at 60°C for 1 hour, and a resultant product was passed through a JIS standard sieve having a mesh size of 710 $\mu$m. Then, 0.4 parts by mass of silicon dioxide (product name: Aerosil 200, manufactured by Nippon Aerosil Co., Ltd.) was added, and a resultant mixture was mixed until uniform.

Through the above operations, a particulate water-absorbing agent (1) was obtained. The physical properties of the particulate water-absorbing agent (1) are indicated in Table 1.

[Production Example 1-2]

In the surface-crosslinking step of Production Example 1, the heating treatment was carried out at 190°C for approximately 60 minutes, so that resultant water-absorbing resin particles (1-2) would have a CRC of 30 g/g. Other steps were carried out in the same manner as in Production Example 1. A particulate water-absorbing agent (1-2) was obtained. The physical properties of the particulate water-absorbing agent (1-2) are indicated in Table 1.

[Production Example 2]

First, there was prepared an aqueous monomer solution (2) consisting of 300 parts by mass of acrylic acid, 100 parts by mass of a 48 mass% aqueous sodium hydroxide solution, 0.61 parts by mass of polyethylene glycol diacrylate (average n number: 9), 6.5 parts by mass of a 1.0 mass% aqueous pentasodium ethylenediamine tetra(methylene phosphonate) solution, and 346.1 parts by mass of deionized water.

Next, 150.6 parts by mass of a 48 mass% aqueous sodium hydroxide solution was mixed with the aqueous monomer solution (2), whose temperature had been adjusted to 40°C.

Next, 14.6 parts by mass of a 4 mass% aqueous sodium persulfate solution was added. Thereafter, the aqueous monomer solution (2) was introduced into a planar polymerization device in a manner so as to have a thickness of 10 mm. Thereafter, polymerization was allowed to proceed (polymerization time: 3 minutes), so that a hydrogel (2) was obtained.

The hydrogel (2) thus obtained had a moisture content of 51.9 mass%.

The hydrogel (2) thus obtained was gel-crushed with use of a screw extruder, so that a particulate hydrogel (2) was obtained.

The particulate hydrogel (2) had a moisture content of 52.5 mass%, a mass average particle diameter (D50) of 860 $\mu$m, and a logarithmic standard deviation ($\sigma\zeta$) of a particle size distribution of 0.95.

Drying, pulverization, and classification were carried out similarly to Production Example 1, so that a water-absorbing resin powder (2) having a non-uniformly pulverized shape was obtained. The water-absorbing resin powder (2) had a mass average particle diameter (D50) of 355 $\mu$m, a logarithmic standard deviation ($\sigma\zeta$) of a particle size distribution of 0.32, a CRC of 48.2 g/g, and a proportion of particles having a particle diameter of less than 150 $\mu$m (a proportion of particles that can pass through a sieve having a mesh size of 150 $\mu$m) of 0.4 mass%.

To 100 parts by mass of the water-absorbing resin powder (2), a surface-crosslinking agent solution (2) consisting of 0.02 parts by mass of ethylene glycol diglycidyl ether, 0.4 parts by mass of ethylene carbonate, 0.6 parts by mass of propylene glycol, and 3.0 parts by mass of deionized water was added. Then, a resultant mixture was mixed until uniform. Thereafter, the mixture was heat-treated at 190°C for approximately 30 minutes so that resulting water-absorbing resin particles (2) would have a CRC of 38 g/g. Then, the mixture was force-cooled to 60°C.

Thereafter, operations similar to those carried out in Production Example 1 were carried out, so that a particulate water-absorbing agent (2) was obtained. The physical properties of the particulate water-absorbing agent (2) are indicated in Table 1.

[Production Example 3]

First, there was prepared an aqueous monomer solution (3) consisting of 300 parts by mass of acrylic acid, 100 parts by mass of a 48 mass% aqueous sodium hydroxide solution, 0.61 parts by mass of polyethylene glycol diacrylate (average n number: 9), 6.5 parts by mass of a 1.0 mass% aqueous pentasodium ethylenediamine tetra(methylene phosphonate) solution, and 371.6 parts by mass of deionized water.

The aqueous monomer solution (3) was polymerized and then subjected to gel-crushing, in the same manner as in Production Example 1.

The particulate hydrogel (3) obtained had a moisture content of 53.4 mass%, a mass average particle diameter (D50) of 627 $\mu$m, and a logarithmic standard deviation ($\sigma\zeta$) of a particle size distribution of 1.02.

The particulate hydrogel (3) was dried in the same manner as in Production Example 1, so that a dried polymer (3) was obtained.

The dried polymer (3) was pulverized and then classified with use of a JIS standard sieve having a mesh size of 425 $\mu$m, so that water-absorbing resin particles (3) having a non-uniformly pulverized shape were obtained. The water-absorbing resin particles (3) had a mass average particle diameter (D50) of 175 $\mu$m, a logarithmic standard deviation ($\sigma\zeta$) of a particle size distribution of 0.56, a CRC of 48.9 g/g, and a proportion of 150 $\mu$m passing particles (a proportion of particles that can pass through a sieve having a mesh size of 150 $\mu$m) of 39 mass%.

With 100 parts by mass of the water-absorbing resin particles (3), a covalent bonding surface-crosslinking agent solution consisting of 0.04 parts by mass of ethylene glycol diglycidyl ether, 1.5 parts by mass of propylene glycol, and 3.5 parts by mass of deionized water was uniformly mixed. Thereafter, a heating treatment and cooling were carried out such that a resultant water-absorbing resin powder (3) would have a CRC of 35 g/g.

The water-absorbing resin particles (3) obtained via the above operations were subjected to a paint shaker test so as to be subjected to damage equivalent to that occurring in a production process. Thereafter, with 100 parts by mass of the water-absorbing resin particles, an aqueous solution consisting of 1 part by mass of water and 0.01 parts by mass of trisodium diethylenetriamine pentaacetate was uniformly mixed. The resulting mixture was dried at 60°C for 1 hour and subsequently passed through a JIS standard sieve having a mesh size of 710 $\mu$m. Thereafter, 0.3 parts by mass of hydrotalcite (DHT-6, manufactured by Kyowa Chemical Industry Co., Ltd.) was uniformly mixed with the substance that passed through the standard sieve. Thus, a particulate water-absorbing agent (3) was obtained. The physical properties of the particulate water-absorbing agent (3) are indicated in Table 1.

[Production Example 4]

First, 1.7 g of trimethylolpropane triacrylate (molecular weight: 296) was dissolved into 5500 g of an aqueous sodium acrylate solution (neutralization rate: 75 mol%, monomer concentration: 38 mass%), so that a reaction solution was obtained. The reaction solution was degassed in a nitrogen gas atmosphere for 30 minutes. Thereafter, the reaction solution was introduced into a reactor (formed by providing a lid to a twin-armed jacketed stainless steel kneader having two sigma type blades and a capacity of 10L). While maintaining the temperature of the reaction solution at 30°C, nitrogen substitution was carried out by blowing nitrogen gas into the reactor, so that the dissolved oxygen concentration in the system was 1 ppm or less.

Subsequently, while stirring the reaction solution, 29.8 g of a 10 mass% aqueous sodium persulfate solution and 21.8 g of a 0.2 mass% aqueous L-ascorbic acid solution were added. As a result, polymerization started after about 1 minute. The temperature in the system reached a peak polymerization temperature of 86°C 17 minutes after the start of polymerization. A hydrogel was removed 60 minutes after the start of the polymerization.

The hydrogel obtained was in a grain-refined state, with particles of approximately 1 mm to 5 mm in size. The grain-refined hydrogel was spread on a 50-mesh (mesh size: 300 μm) metal gauze and hot-air dried at 180°C for 45 minutes, so that a dried material was obtained.

The dried material was then pulverized with use of a roll mill and subsequently classified continuously using a metal gauze having a mesh size of 450 μm and a metal gauze having a mesh size of 106 μm. The particles that remained on the metal gauze having a mesh size of 450 μm were again subjected to pulverization by a roll mill. Water-absorbing resin fine particles which passed through the metal gauze having the mesh size of 106 μm were mixed with an equal amount of water that had been heated to 90°C, dried again under the same conditions, and then pulverized (the amount of these water-absorbing resin fine particles was 13 mass% relative to the total amount of the dried material subjected to pulverization). In this way, water-absorbing resin particles (4) having a non-uniformly pulverized shape were obtained. The water-absorbing resin particles (4) had a CRC of 44.5 g/g.

With 100 parts by mass of the water-absorbing resin particles (4), 3.5 parts by mass of an aqueous surface-crosslinking agent solution were mixed, the aqueous surface-crosslinking agent solution consisting of 0.5 parts by mass of glycerin, 0.5 parts by mass of isopropyl alcohol, and 2.5 parts by mass of deionized water. The resulting mixture was subjected to a heating treatment at 190°C for 40 minutes, so that a water-absorbing resin powder (4) was obtained.

Further, to 100 parts by mass of the water-absorbing resin powder (4), 0.3 parts by mass of silicon dioxide (Aerosil 200, manufactured by Aerosil, Japan) was added and mixed uniformly. In this way, a particulate water-absorbing agent (4) was obtained. The physical properties of the particulate water-absorbing agent (4) are indicated in Table 1.

[Removal 1 of Water-Absorbing Resin from Commercially Available Disposable Diaper]

A water-absorbing resin was removed from a commercially available disposable diaper (Moony Air Fit (size L, Lot No.201512163072), manufactured by Unicharm, purchased in May 2016). During removal, only the water-absorbing resin was taken out so that e.g. cotton-like pulp was not mixed therewith. The water-absorbing resin thus removed was in the form of a granulated material of spherical particles. This water-absorbing resin was referred to as a particulate water-absorbing agent (T1). The physical properties of the particulate water-absorbing agent (T1) are indicated in Table 1.

[Removal 2 of Water-Absorbing Resin from Commercially Available Disposable Diaper]

A water-absorbing resin was removed from a commercially available disposable diaper (HUGGIES (4 Maxi size, Lot No. EXP30/04/19), manufactured by Kimberly-Clark, purchased in Turkey in June 2016). During removal, only the water-absorbing resin was taken out so that e.g. cotton-like pulp was not mixed therewith. The water-absorbing resin thus removed was in the form of crushed particles. This water-absorbing resin was referred to as a particulate water-absorbing agent (T2). The physical properties of the particulate water-absorbing agent (T2) are indicated in Table 1.

[Production Example 5]

The particulate water-absorbing agent (2) and the particulate water-absorbing agent (T1) were mixed. The ratio at which these substances were mixed was changed so as to produce a particulate water-absorbing agent (5-1) having a GPR of 38 g/min, a particulate water-absorbing agent (5-2) having a GPR of 27 g/min, and a particulate water-absorbing agent (5-3) having a GPR of 19 g/min.

[Production Examples 6 to 8]

With reference to the Production Examples, Examples, and Comparative Examples described in the patents below, polyacrylic acid (salt)-based resin particulate water-absorbing agents (6) to (8) were obtained by, e.g., adjusting CRC as appropriate by adjustment of the amount of an internal crosslinking agent. The particulate water-absorbing agent (6) had an FSCw of 32 g/g. The particulate water-absorbing agent (7) had an FSCw of 44 g/g. The particulate water-absorbing agent (8) had an FSCw of 50 g/g. The physical properties of the particulate water-absorbing agents obtained are indicated in Table 1.

International Publication No. WO 2014/034897
International Publication No. WO 2017/170605
International Publication No. WO 2016/204302
International Publication No. WO 2014/054656
International Publication No. WO 2015/152299
International Publication No. WO 2018/062539
International Publication No. WO 2012/043821

[Production Example 9]

The particulate water-absorbing agent (6) and the particulate water-absorbing agent (T1) were mixed. The ratio at which the substances were mixed was changed so as to produce a particulate water-absorbing agent (9) having a GPR of 21 g/min.

[Production Example 10]

The particulate water-absorbing agent (7) and the particulate water-absorbing agent (T1) were mixed. The ratio at which the substances were mixed was changed so as to produce a particulate water-absorbing agent (10) having a GPR of 5 g/min.

[Production Example 11]

The particulate water-absorbing agent (8) and the particulate water-absorbing agent (T1) were mixed. The ratio at which the substances were mixed was changed so as to produce a particulate water-absorbing agent (11) having a GPR of 3 g/min.

[Table 1]

| | | CRC [g/g] | Gel permeation rate GPR [g/min] | Surface tension [mN/m] |
|---|---|---|---|---|
| Prod. Ex. 1 | Part. water-abs. agent (1) | 33 | 157 | 72 |
| Prod. Ex. 1-2 | Part. water-abs. agent (1-2) | 30 | 201 | 72 |
| Prod. Ex. 2 | Part. water-abs. agent (2) | 38 | 51 | 72 |
| Prod. Ex. 3 | Part. water-abs. agent (3) | 35 | 4 | 72 |
| Prod. Ex. 4 | Part. water-abs. agent (4) | 36 | 0 | 72 |
| Removal | Part. water-abs. agent (T1) | 35 | 2 | 58 |
| Removal | Part. water-abs. agent (T2) | 30 | 91 | 55 |
| Prod. Ex. 5 | Part. water-abs. agent (5-1) | 38 | 38 | - |
| Prod. Ex. 5 | Part. water-abs. agent (5-2) | 37 | 27 | - |
| Prod. Ex. 5 | Part. water-abs. agent (5-3) | 36 | 19 | - |
| Prod. Ex. 6 | Part. water-abs. agent (6) | 40 | 40 | 72 |
| Prod. Ex. 7 | Part. water-abs. agent (7) | 38 | 7 | 72 |
| Prod. Ex. 8 | Part. water-abs. agent (8) | 38 | 4 | 72 |

(continued)

|  |  | CRC [g/g] | Gel permeation rate GPR [g/min] | Surface tension [mN/m] |
|---|---|---|---|---|
| Prod. Ex. 9 | Part. water-abs. agent (9) | 38 | 21 | - |
| Prod. Ex. 10 | Part. water-abs. agent (10) | 37 | 5 | - |
| Prod. Ex. 11 | Part. water-abs. agent (11) | 37 | 3 | - |
| Note: In Table 1, "Prod. Ex." stands for "Production Example", and "Part. water-abs. agent" stands for "Particulate water-absorbing agent". | | | | |

[Example 1]

First, 0.1 g to 0.2 g of an adhesive ("Spray Nori 77", manufactured by 3M Japan Limited) containing styrene butadiene rubber was dispersed (dispersion amount: 7.8 g/m$^2$ to 15.6 g/m$^2$) onto a surface of a pulp fiber nonwoven fabric (1) (corresponding to the second sheet; mass per unit area: 42 g/m$^2$) which had been cut to a size of 8 cm by 16 cm.

Next, 1.405 g of the particulate water-absorbing agent (3) (corresponding to the second particulate water-absorbing agent) obtained in Production Example 3 was uniformly dispersed (dispersion amount: 110 g/m$^2$) onto the pulp fiber nonwoven fabric (1) onto which the adhesive had been dispersed. A polypropylene nonwoven fabric (2) (corresponding to the intermediate sheet; mass per unit area: 50.6 g/m$^2$; having a degree of thickness, the thickness being approximately 4 mm to 6 mm without load) was placed thereon, and pressure bonding was carried out.

Next, 0.1 g to 0.2 g of an adhesive ("Spray Nori 77", manufactured by 3M Japan Limited) containing styrene butadiene rubber was dispersed (dispersion amount: 7.8 g/m$^2$ to 15.6 g/m$^2$) onto a surface of the polypropylene nonwoven fabric (2) on a side not facing the particulate water-absorbing agent (3). Next, 1.405 g of the particulate water-absorbing agent (1) (corresponding to the first particulate water-absorbing agent) obtained in Production Example 1 was uniformly dispersed thereon (dispersion amount: 110 g/m$^2$).

Finally, a pulp fiber nonwoven fabric (1) (corresponding to the first sheet; mass per unit area: 15 g/m$^2$) was placed on the particulate water-absorbing agent (1), and pressure bonding was carried out. In this way a water-absorbing sheet (1) was obtained.

Note that the pulp fiber nonwoven fabrics (1) and the polypropylene nonwoven fabric (2) used in the present Example are both water-permeable sheets.

[Example 2]

A water-absorbing sheet (2) was obtained in a manner similar to Example 1, except that the particulate water-absorbing agent (4) was used instead of the particulate water-absorbing agent (3) as the second particulate water-absorbing agent.

[Example 3]

A water-absorbing sheet (3) was obtained in a manner similar to Example 1, except that the particulate water-absorbing agent (2) was used instead of the particulate water-absorbing agent (1) as the first particulate water-absorbing agent.

[Example 4]

A water-absorbing sheet (4) was obtained in a manner similar to Example 1, except that the particulate water-absorbing agent (1-2) was used instead of the particulate water-absorbing agent (1) as the first particulate water-absorbing agent.

[Example 5]

A water-absorbing sheet (5) was obtained in a manner similar to Example 1, except that the particulate water-absorbing agent (T1) was used instead of the particulate water-absorbing agent (3) as the second particulate water-absorbing agent.

[Example 6]

A water-absorbing sheet (6) was obtained in a manner similar to Example 1, except that the particulate water-absorbing agent (5-1) was used instead of the particulate water-absorbing agent (3) as the second particulate water-absorbing agent.

[Example 7]

A water-absorbing sheet (7) was obtained in a manner similar to Example 1, except that the particulate water-absorbing agent (5-2) was used instead of the particulate water-absorbing agent (3) as the second particulate water-absorbing agent.

[Example 8]

A water-absorbing sheet (8) was obtained in a manner similar to Example 1, except that the particulate water-absorbing agent (5-3) was used instead of the particulate water-absorbing agent (3) as the second particulate water-absorbing agent.

[Example 9]

A water-absorbing sheet (9) was obtained in a manner similar to Example 1, except that the particulate water-absorbing agent (2) was used instead of the particulate water-absorbing agent (1) as the first particulate water-absorbing agent, and the particulate water-absorbing agent (5-3) was used instead of the particulate water-absorbing agent (3) as the second particulate water-absorbing agent.

[Example 10]

A water-absorbing sheet (10) was obtained in a manner similar to Example 1, except that the particulate water-absorbing agent (9) was used instead of the particulate water-absorbing agent (3) as the second particulate water-absorbing agent.

[Example 11]

A water-absorbing sheet (11) was obtained in a manner similar to Example 1, except that the particulate water-absorbing agent (10) was used instead of the particulate water-absorbing agent (3) as the second particulate water-absorbing agent.

[Example 12]

A water-absorbing sheet (12) was obtained in a manner similar to Example 1, except that the particulate water-absorbing agent (11) was used instead of the particulate water-absorbing agent (3) as the second particulate water-absorbing agent.

[Comparative Example 1]

A comparative water-absorbing sheet (1) was obtained in a manner similar to Example 1, except that the particulate water-absorbing agent (1) was used instead of the particulate water-absorbing agent (3) as the second particulate water-absorbing agent.

[Comparative Example 2]

A comparative water-absorbing sheet (2) was obtained in a manner similar to Example 1, except that the particulate water-absorbing agent (2) was used instead of the particulate water-absorbing agents (1) and (3) (i.e., the particulate water-absorbing agent (2) was used as both the first particulate water-absorbing agent and the second particulate water-absorbing agent).

[Comparative Example 3]

A comparative water-absorbing sheet (3) was obtained in a manner similar to Example 1, except that the particulate water-absorbing agent (2) was used instead of the particulate water-absorbing agent (3) as the second particulate water-absorbing agent.

[Comparative Example 4]

A comparative water-absorbing sheet (4) was obtained in a manner similar to Example 1, except that the particulate water-absorbing agent (3) was used instead of the particulate water-absorbing agent (1) as the first particulate water-absorbing agent.

[Comparative Example 5]

A comparative water-absorbing sheet (5) was obtained in a manner similar to Example 1, except that the particulate water-absorbing agent (3) was used instead of the particulate water-absorbing agent (1) as the first particulate water-absorbing agent, and the particulate water-absorbing agent (T1) was used instead of the particulate water-absorbing agent (3) as the second particulate water-absorbing agent.

[Comparative Example 6]

A comparative water-absorbing sheet (6) was obtained in a manner similar to Example 1, except that the particulate water-absorbing agent (T2) was used instead of the particulate water-absorbing agent (1) as the first particulate water-absorbing agent, and the particulate water-absorbing agent (T2) was used instead of the particulate water-absorbing agent (3) as the second particulate water-absorbing agent.

(Results)

Each of the water-absorbing sheets (1) to (9) and the comparative water-absorbing sheets (1) to (6) were subjected to water-absorbing sheet evaluation in a flat state as described above. Table 2 indicates flat-state surface liquid absorption speed and flat-state re-wet.

[Table 2]

| | First part. water-abs. agent GPR [g/min] | Second part. water-abs. agent GPR [g/min] | ΔGPR [g/min] | Flat state evaluation of water-abs. sheet | |
| --- | --- | --- | --- | --- | --- |
| | | | | Flat-state surface liquid abs. speed (Liquid abs. time) [sec] | Flat-state re-wet [g] |
| Ex. 1 | 157 | 4 | 153 | 25 | 0.7 |
| Ex. 2 | 157 | 0 | 157 | 40 | 1.4 |
| Ex. 3 | 51 | 4 | 47 | 27 | 1.1 |
| Ex. 4 | 201 | 4 | 197 | 25 | 0.8 |
| Ex. 5 | 157 | 2 | 155 | 33 | 0.8 |
| Ex. 6 | 157 | 38 | 119 | 35 | 1.2 |
| Ex. 7 | 157 | 27 | 130 | 40 | 1.5 |
| Ex. 8 | 157 | 19 | 138 | 43 | 1.7 |
| Ex. 9 | 51 | 19 | 32 | 45 | 1.7 |
| Ex. 10 | 157 | 21 | 136 | 46 | 1.3 |
| Ex. 11 | 157 | 5 | 152 | 41 | 1.5 |
| Ex. 12 | 157 | 3 | 154 | 36 | 1.2 |
| Comp. Ex. 1 | 157 | 157 | 0 | 55 | 2.3 |
| Comp. Ex. 2 | 51 | 51 | 0 | 52 | 2.0 |

(continued)

| | First part. water-abs. agent GPR [g/min] | Second part. water-abs. agent GPR [g/min] | ΔGPR [g/min] | Flat state evaluation of water-abs. sheet | |
| --- | --- | --- | --- | --- | --- |
| | | | | Flat-state surface liquid abs. speed (Liquid abs. time) [sec] | Flat-state re-wet [g] |
| Comp. Ex. 3 | 157 | 51 | 106 | 50 | 2.1 |
| Comp. Ex. 4 | 4 | 4 | 0 | 21 | 2.8 |
| Comp. Ex. 5 | 4 | 2 | 2 | 24 | 2.5 |
| Comp. Ex. 6 | 91 | 91 | 0 | 41 | 2.5 |
| Note: In Table 2, "Ex." stands for "Example", "Comp. Ex." stands for "Comparative Example", "part. water-abs. agent" stands for "particulate water-absorbing agent", "GPR" stands for "gel permeation rate", "water-abs. sheet" stands for "water-absorbing sheet", and "liquid abs." stands for "liquid absorption". | | | | | |

As indicated in Table 2, the water-absorbing sheets (1) to (9) obtained in Examples 1 to 9 were favorable in terms of both liquid absorption speed and re-wet. In particular, considering the balance between these properties, the water-absorbing sheets (1), (3), (4), and (5) exhibited more favorable properties, and the water-absorbing sheets (1), (4), and (5) exhibited particularly favorable properties.

In a comparison between Example 1 and Comparative Example 1, and a comparison between Example 2 and Comparative Example 2, it can be seen that a configuration in which there is a difference in the gel permeation rate (GPR) between the first particulate water-absorbing agent and the second particulate water-absorbing agent makes it possible to obtain a water-absorbing sheet that is favorable in terms of both liquid absorption speed and re-wet. By comparing Examples 1 to 9 to Comparative Example 5, it can also be understood that if the difference in the gel permeation rate (GPR) is not large enough, the above effect cannot be obtained.

Further, by comparing Examples 1 and 2 to Comparative Example 3, it can be seen that, if the gel permeation rate (GPR) of the second particulate water-absorbing agent is too high, it is impossible to obtain a water-absorbing sheet that is favorable in terms of both liquid absorption speed and re-wet.

In Examples 6 to 12, the particulate water-absorbing agents (5-1) to (5-3) and (9) to (12) were used. These are the particulate water-absorbing agents whose gel permeation rates (GPRs) was adjusted by mixing a plurality of types of particulate water-absorbing agents. It can be seen that an effect of the present invention is brought about even in a case where such a particulate water-absorbing agent is used.

Industrial Applicability

A water-absorbing sheet in accordance with an embodiment of the present invention has various applications such as applications in hygienic materials (such as disposable diapers and sanitary napkins), sheets for pets, and waterproofing agents.

Reference Signs List

10: Water-absorbing sheet
11a: First sheet
11b: Second sheet
12: Particulate water-absorbing agent
12a: First particulate water-absorbing agent (particulate water-absorbing agent A)
12b: Second particulate water-absorbing agent (particulate water-absorbing agent B)
13: Intermediate sheet
14: Adhesive

Claims

1.    A water-absorbing sheet (10) comprising:

a first sheet (11a);
a second sheet (11b); and
a particulate water-absorbing agent (12) sandwiched between said first sheet (11a) and said second sheet (11b),
at least one sheet selected from said first sheet (11a) and said second sheet (11b) being a water-permeable sheet,
said particulate water-absorbing agent (12) including:

(1) a first particulate water-absorbing agent (12a) localized in the vicinity of said first sheet (11a); and
(2) a second particulate water-absorbing agent (12b) localized in the vicinity of said second sheet (11b),

said first particulate water-absorbing agent (12a) having a gel permeation rate (GPR) which is higher than that of said second particulate water-absorbing agent (12b),
the gel permeation rate (GPR) of said first particulate water-absorbing agent (12a) differing by 10 g/min or more from that of said second particulate water-absorbing agent (12b),
the gel permeation rate (GPR) of said second particulate water-absorbing agent (12b) being 50 g/min or less,
wherein the gel permeation rate is measured according to the method as disclosed in the description.

2. The water-absorbing sheet (10) according to claim 1, wherein at least one selected from said first particulate water-absorbing agent (12a) and said second particulate water-absorbing agent (12b) includes a particulate water-absorbing agent having a surface tension of 65 mN/m or more.

3. The water-absorbing sheet (10) according to claim 1 or 2, wherein at least one selected from said first particulate water-absorbing agent (12a) and said second particulate water-absorbing agent (12b) includes a particulate water-absorbing agent having a non-uniformly pulverized shape.

4. The water-absorbing sheet (10) according to any one of claims 1 to 3, further comprising one or more intermediate sheets (13) sandwiched between said first sheet (11a) and said second sheet (11b).

5. The water-absorbing sheet (10) according to any one of claims 1 to 4, wherein said first sheet (11a) is the water-permeable sheet.

6. The water-absorbing sheet (10) according to any one of claims 1 to 5, wherein the water-permeable sheet is a hydrophilic nonwoven fabric.

7. The water-absorbing sheet (10) according to any one of claims 1 to 6, wherein the water-absorbing sheet has a region in which at least one selected from said first particulate water-absorbing agent (12a) and said second particulate water-absorbing agent (12b) is absent, the region extending in a lengthwise direction of the water-absorbing sheet (10).

8. The water-absorbing sheet (10) according to any one of claims 1 to 7, wherein the water-absorbing sheet (10) has a thickness, in a dry state, of 5 mm or less.

9. A method of producing the water-absorbing sheet (10) according to any one of claims 1 to 8, comprising at least one of the following steps:

(1) dispersing a first particulate water-absorbing agent (12a) onto a first sheet (11a);
(2) dispersing a second particulate water-absorbing agent (12b) onto a second sheet (11b); and
(3) dispersing said first particulate water-absorbing agent (12a) and/or said second particulate water-absorbing agent (12b) onto an intermediate sheet (13),

at least one sheet selected from said first sheet (11a) and said second sheet (11b) being a water-permeable sheet,
said first particulate water-absorbing agent (12a) having a gel permeation rate (GPR) which is higher than that of said second particulate water-absorbing agent (12b),
the gel permeation rate (GPR) of said first particulate water-absorbing agent (12a) differing by 10 g/min or more from that of said second particulate water-absorbing agent (12b),
the gel permeation rate (GPR) of said second particulate water-absorbing agent (12b) being 50 g/min or less,
wherein the gel permeation rate is measured according to the method as disclosed in the description.

**10.** An absorbent article comprising:

a liquid-permeable sheet;
a liquid-impermeable sheet; and
the water-absorbing sheet (10) according to any one of claims 1 to 8, which is sandwiched between the liquid-permeable sheet and the liquid-impermeable sheet.

**11.** The absorbent article according to claim 10, wherein:

said first sheet (11a) is the water-permeable sheet; and
said first sheet (11a) is provided on a side toward said liquid-permeable sheet.

**Patentansprüche**

**1.** Wasserabsorbierendes Sheet (10), das folgendes umfasst:

ein erstes Sheet (11a);
ein zweites Sheet (11b); und
ein partikelförmiges wasserabsorbierendes Mittel (12), das sich zwischen dem ersten Sheet (11a) und dem zweiten Sheet (11b) befindet,
wobei mindestens ein Sheet, ausgewählt aus dem ersten Sheet (11a) und dem zweiten Sheet (11b), ein wasserdurchlässiges Sheet ist,
wobei das partikelförmige wasserabsorbierende Mittel (12) einschließt:

(1) ein erstes partikelförmiges wasserabsorbierendes Mittel (12a), das sich in der Nähe des ersten Sheets (11a) befindet; **und**
(2) ein zweites partikelförmiges wasserabsorbierendes Mittel (12b), das sich in der Nähe des zweiten Sheets (11b) befindet,

wobei das erste partikelförmige wasserabsorbierende Mittel (12a) eine Gelpermeationsrate (GPR) besitzt, die höher ist als diejenige des zweiten partikelförmigen wasserabsorbierenden Mittels (12b),
die Gelpermeationsrate (GPR) des ersten partikelförmigen wasserabsorbierenden Mittels (12a) sich um 10 g/min oder mehr von derjenigen des zweiten partikelförmigen wasserabsorbierenden Mittels (12b) unterscheidet und
die Gelpermeationsrate (GPR) des zweiten partikelförmigen wasserabsorbierenden Mittels (12b) 50 g/min oder weniger ist,
wobei die Gelpermeationsrate nach der in der Beschreibung offenbarten Methode gemessen wird.

**2.** Wasserabsorbierendes Sheet (10) gemäß Anspruch 1, in dem mindestens eines, ausgewählt aus dem ersten partikelförmigen wasserabsorbierenden Mittel (12a) und dem zweiten partikelförmigen wasserabsorbierenden Mittel (12b), ein partikelförmiges wasserabsorbierendes Mittel mit einer Oberflächenspannung von 65 mN/m oder mehr einschließt.

**3.** Wasserabsorbierendes Sheet (10) gemäß Anspruch 1 oder 2, in dem mindestens eines, ausgewählt aus dem ersten partikelförmigen wasserabsorbierenden Mittel (12a) und dem zweiten partikelförmigen wasserabsorbierenden Mittel (12b), ein partikelförmiges wasserabsorbierendes Mittel mit einer nicht gleichförmigen pulverisierten Form einschließt.

**4.** Wasserabsorbierendes Sheet (10) gemäß mindestens einem der Ansprüche 1 bis 3, das ferner eines oder mehrere Zwischensheets (13), die sich zwischen dem ersten Sheet (11a) und dem zweiten Sheet (11b) befinden, umfasst.

**5.** Wasserabsorbierendes Sheet (10) gemäß mindestens einem der Ansprüche 1 bis 4, in dem das erste Sheet (11a) das wasserdurchlässige Sheet ist.

**6.** Wasserabsorbierendes Sheet (10) gemäß mindestens einem der Ansprüche 1 bis 5, in dem das wasserdurchlässige Sheet ein hydrophiles Vliesstoffgewebe ist.

7. Wasserabsorbierendes Sheet (10) gemäß mindestens einem der Ansprüche 1 bis 6, wobei das wasserabsorbierende Sheet einen Bereich aufweist, in dem mindestens eines, ausgewählt aus dem ersten partikelförmigen wasserabsorbierenden Mittel (12a) und dem zweiten partikelförmigen wasserabsorbierenden Mittel (12b), abwesend ist, wobei sich der Bereich in einer Längsrichtung des wasserabsorbierenden Sheets (10) erstreckt.

8. Wasserabsorbierendes Sheet (10) gemäß mindestens einem der Ansprüche 1 bis 7, wobei das wasserabsorbierende Sheet (10) in trockenem Zustand eine Dicke von 5 mm oder weniger besitzt.

9. Verfahren zur Herstellung des wasserabsorbierenden Sheets (10) gemäß mindestens einem der Ansprüche 1 bis 8, das mindestens einen der folgenden Schritte umfasst:

(1) Dispergieren eines ersten partikelförmigen wasserabsorbierenden Mittels (12a) auf ein erstes Sheet (11a);
(2) Dispergieren eines zweiten partikelförmigen wasserabsorbierenden Mittels (12b) auf ein zweites Sheet (11b); und
(3) Dispergieren des ersten partikelförmigen wasserabsorbierenden Mittels (12a) und/oder des zweiten partikelförmigen wasserabsorbierenden Mittels (12b) auf ein Zwischensheet (13),
wobei mindestens ein Sheet, ausgewählt aus dem ersten Sheet (11a) und dem zweiten Sheet (11b), ein wasserdurchlässiges Sheet ist,
das erste partikelförmige wasserabsorbierende Mittel (12a) eine Gelpermeationsrate (GPR) besitzt, die höher ist als diejenige des zweiten partikelförmigen wasserabsorbierenden Mittels (12b),
die Gelpermeationsrate (GPR) des ersten partikelförmigen wasserabsorbierenden Mittels (12a) sich um 10 g/min oder mehr von derjenigen des zweiten partikelförmigen wasserabsorbierenden Mittels (12b) unterscheidet **und**
die Gelpermeationsrate (GPR) des zweiten partikelförmigen wasserabsorbierenden Mittels (12b) 50 g/min oder weniger ist,
wobei die Gelpermeationsrate nach der in der Beschreibung offenbarten Methode gemessen wird.

10. Absorbierender Artikel, der folgendes umfasst:

ein flüssigkeitsdurchlässiges Sheet;
ein flüssigkeitsundurchlässiges Sheet; und
das wasserabsorbierende Sheet (10) gemäß mindestens einem der Ansprüche 1 bis 8, das sich zwischen dem flüssigkeitsdurchlässigen Sheet und dem flüssigkeitsundurchlässigen Sheet befindet.

11. Absorbierender Artikel gemäß Anspruch 10, in dem:

das erste Sheet (11a) das wasserdurchlässige Sheet ist; und
das erste Sheet (11a) auf einer Seite in Richtung des flüssigkeitsdurchlässigen Sheets (11a) vorgesehen ist.

**Revendications**

1. Feuille absorbant l'eau (10) comprenant :

une première feuille (11a) ;
une seconde feuille (11b) ; et
un agent absorbant l'eau particulaire (12) pris en sandwich entre ladite première feuille (11a) et ladite seconde feuille (11b),
au moins une feuille sélectionnée parmi ladite première feuille (11a) et ladite seconde feuille (11b) étant une feuille perméable à l'eau,
ledit agent absorbant l'eau particulaire (12) incluant :

(1) un premier agent absorbant l'eau particulaire (12a) localisé au voisinage de ladite première feuille (11a) ; et
(2) un second agent absorbant l'eau particulaire (12b) localisé au voisinage de ladite seconde feuille (11b),

ledit premier agent absorbant l'eau particulaire (12a) présentant un taux de perméation de gel (GPR) qui est supérieur à celui dudit second agent absorbant l'eau particulaire (12b),

EP 3 777 803 B1

le taux de perméation de gel (GPR) dudit premier agent absorbant l'eau particulaire (12a) différant de 10 g/min ou plus de celui dudit second agent absorbant l'eau particulaire (12b),
le taux de perméation de gel (GPR) dudit second agent absorbant l'eau particulaire (12b) étant 50 g/min ou moins,
dans laquelle le taux de perméation de gel est mesuré selon le procédé tel qu'énoncé dans la description.

2. Feuille absorbant l'eau (10) selon la revendication 1, dans laquelle au moins un sélectionné parmi ledit premier agent absorbant l'eau particulaire (12a) et ledit second agent absorbant l'eau particulaire (12b) inclut un agent absorbant l'eau particulaire présentant une tension de surface de 65 mN/m ou plus.

3. Feuille absorbant l'eau (10) selon la revendication 1 ou 2, dans laquelle au moins un sélectionné parmi ledit premier agent absorbant l'eau particulaire (12a) et ledit second agent absorbant l'eau particulaire (12b) inclut un agent absorbant l'eau particulaire présentant une forme non uniformément pulvérisée.

4. Feuille absorbant l'eau (10) selon l'une quelconque des revendications 1 à 3, comprenant en outre une ou plusieurs feuilles intermédiaires (13) prises en sandwich entre ladite première feuille (11a) et ladite seconde feuille (11b).

5. Feuille absorbant l'eau (10) selon l'une quelconque des revendications 1 à 4, dans laquelle ladite première feuille (11a) est la feuille perméable à l'eau.

6. Feuille absorbant l'eau (10) selon l'une quelconque des revendications 1 à 5, dans laquelle la feuille perméable à l'eau est un tissu non tissé hydrophile.

7. Feuille absorbant l'eau (10) selon l'une quelconque des revendications 1 à 6, dans laquelle la feuille absorbant l'eau présente une région dans laquelle au moins un sélectionné parmi ledit premier agent absorbant l'eau particulaire (12a) et ledit second agent absorbant l'eau particulaire (12b) est absent, la région s'étendant dans une direction de longueur de la feuille absorbant l'eau (10).

8. Feuille absorbant l'eau (10) selon l'une quelconque des revendications 1 à 7, dans laquelle la feuille absorbant l'eau (10) présente une épaisseur, dans un état sec, de 5 mm ou moins.

9. Procédé de production de la feuille absorbant l'eau (10) selon l'une quelconque des revendications 1 à 8, comprenant au moins l'une des étapes suivantes :

(1) la dispersion d'un premier agent absorbant l'eau particulaire (12a) sur une première feuille (11a) ;
(2) la dispersion d'un second agent absorbant l'eau particulaire (12b) sur une seconde feuille (11b) ; et
(3) la dispersion dudit premier agent absorbant l'eau particulaire (12a) et/ou dudit second agent absorbant l'eau particulaire (12b) sur une feuille intermédiaire (13),
au moins une feuille sélectionnée parmi ladite première feuille (11a) et ladite seconde feuille (11b) étant une feuille perméable à l'eau,
ledit premier agent absorbant l'eau particulaire (12a) présentant un taux de perméation de gel (GPR) qui est supérieur à celui dudit second agent absorbant l'eau particulaire (12b),
le taux de perméation de gel (GPR) dudit premier agent absorbant l'eau particulaire (12a) différant de 10 g/min ou plus de celui dudit second agent absorbant l'eau particulaire (12b),
le taux de perméation de gel (GPR) dudit second agent absorbant l'eau particulaire (12b) étant 50 g/min ou moins,
dans lequel le taux de perméation de gel est mesuré selon le procédé tel qu'énoncé dans la description.

10. Article absorbant comprenant :

une feuille perméable aux liquides ;
une feuille imperméable aux liquides ; et
la feuille absorbant l'eau (10) selon l'une quelconque des revendications 1 à 8, qui est prise en sandwich entre la feuille perméable aux liquides et la feuille imperméable aux liquides.

11. Article absorbant selon la revendication 10, dans lequel :

ladite première feuille (11a) est la feuille perméable à l'eau ; et
ladite première feuille (11a) est fournie sur un côté en direction de ladite feuille perméable aux liquides.

34

FIG. 1

FIG. 2

FIG. 3

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010082373 A
- WO 2011086843 A
- WO 2012174026 A
- WO 2013078109 A
- WO 2015041784 A
- WO 2011117187 A
- WO 2012001117 A
- WO 2012024445 A
- WO 2010004894 A
- WO 2010004895 A
- WO 2010076857 A
- WO 2010113754 A
- WO 2010143635 A
- WO 2011043256 A
- WO 2011086841 A
- WO 2011086842 A
- WO 2011086844 A
- WO 2011117997 A
- WO 2011118409 A
- WO 2011136087 A
- WO 2012043546 A
- WO 2013099634 A
- WO 2013099635 A
- JP 2010115406 A
- JP 2002345883 A
- JP 6315501 A
- JP 6190003 A
- JP 6190002 A
- JP 6190001 A
- JP 2252558 A
- JP 2252560 A
- JP 2252561 A
- US 5849405 A
- US 7638570 B
- US 2006204755
- WO 2009123197 A
- US 20080194863
- US 20050215734
- US 6241928 B
- US 7265190 B
- WO 2006100300 A
- WO 2011025012 A
- WO 2011025013 A
- WO 2011111657 A
- US 7183456 B
- WO 2014034897 A
- WO 2017170605 A
- WO 2016204302 A
- WO 2014054656 A
- WO 2015152299 A
- WO 2018062539 A
- WO 2012043821 A

**Non-patent literature cited in the description**

- McGraw-Hill Dictionary of Scientific and Technical Terms. 1996, 1929
- JAPAN TAPPI Paper and Pulp Test Methods. Japan Technical Association of the Paper and Pulp Technical Association, 2000